# EUROPEAN PATENT SPECIFICATION

(11) **EP 2 401 611 B1**
(45) Date of publication and mention of the grant of the patent: **06.11.2013**
(21) Application number: 10706318.2
(22) Date of filing: 26.02.2010
(51) Int. Cl.: G01N 33/50

(54) **IMPROVED METHODS FOR IDENTIFICATION**
VERBESSERTE IDENTIFZIERUNGSVERFAHREN
PROCÉDÉS AMÉLIORÉS D'IDENTIFICATION

(30) Priority: 27.02.2009 GB 0903495; 27.02.2009 US 394897
(43) Date of publication of application: 04.01.2012
(73) Proprietor: Cambridge Enterprise Limited, Trinity Lane Cambridge Cambridgeshire CB2 1TN (GB)
(72) Inventor: GRAINGER, David, John, Cambridge CB22 4RA (GB)
(74) Representative: Roberts, Michael Austin
(86) International application number: PCT/GB2010/000354
(87) International publication number: WO 2010/097600

(56) References cited:
- GRAINGER, D. ET AL: "Broad-spectrum chemokine inhibitors (BSCIs) and their anti-inflammatory effects in vivo" BIOCHEMICAL PHARMACOLOGY, vol. 65, 2003, pages 1027-1034, XP002579387 cited in the application
- GRAINGER D J ET AL: "BROAD SPECTRUM CHEMOKINE INHIBITORS RELATED TO NR58-3.14.3" MINI REVIEWS IN MEDICINAL CHEMISTRY, BENTHAM SCIENCE PUBLISHERS, HILVERSUM, NL LNKD- DOI:10.2174/1389557054867101, vol. 5, no. 9, 2005, pages 825-832, XP009057659 ISSN: 1389-5575 cited in the application
- VIOLA, A. ET AL: "Chemokines and their receptors: drug targets in immunity and inflammation" ANNU. REV. PHARMACOL. TOXICOL., vol. 48, 2008, pages 171-197, XP002579388
- FOX, D. J. ET AL: "Highly potent, orally available anti-inflammatory broad-spectrum chemokine inhibitors" J. MED, CHEM., vol. 52, 2009, pages 3591-3595, XP002579389

## Description

The invention relates to methods for the discovery of new chemical entities and pharmaceutical compositions with broad-spectrum chemokine inhibitor (BSCI) activity, together with the use of such agents as anti-inflammatory medicaments.

Chemokines are a family of almost 50 structurally-related, mostly small (8-12kDa) proteins which play a key role in regulation of the mammalian immune system. The chemokine ligands signal through almost 20 closely-related receptors, which are members of the type-1 class of G-protein Coupled Receptors (GPCRs). Importantly, many of the ligands bind to, and productively signal through, more than one of the receptors. Similarly, many of the receptors bind to more than one of the ligands.

Varying subsets of these chemokine receptors are expressed on particular types of leukocyte. As a result of this complex degeneracy, tight temporal and spatial control of leukocyte recruitment is possible. It is likely that chemokines play a central role as the "traffic police" of the immune system, encoding the complex route that various leukocytes take from the bone marrow, through the blood, to secondary lymphoid tissue or to the site of an inflammatory response.

For this reason, compounds which modulate chemokine signalling have long been thought of as promising candidates for the development of new anti-inflammatory medications, useful in a very wide range of diseases with an inflammatory component (such as atherosclerosis, autoimmune disorders, allergy, eczema, psoriasis or neurodegenerative disorders). There is considerable need for new medications for these diseases: existing small molecule therapies are generally either effective but have severe side-effects (e.g. corticosteroids) or else have only moderate efficacy but are relatively safe (e.g. NSAIDs such as diclofenac). Biological therapeutics (such as antibodies against TNF-α) may be more specific, but are costly to produce and are unsuitable for simple oral administration. Small molecule chemokine inhibitors offer the promise of a better trade-off between efficacy and safety than for other classes of small molecule anti-inflammatory agents, and offer improved pharmaceutical properties, including cost, compared with existing biological anti-inflammatory agents.

Unfortunately, specific chemokine receptor antagonists have been slow to fulfil this promise. Firstly, they have been difficult to find using conventional high-throughput molecular screening techniques (for example, where random libraries are screened in receptor binding assays for agents capable of blocking the ligand:receptor interaction). The similarity between chemokine receptors has meant that compounds often react with a subset of chemokine receptors (rather than having specificity for a single receptor), and worse still many early chemokine receptor modulating agents bound to related receptors in the serotonin and catecholamine systems resulting in undesirable side-effects on behaviour, blood pressure and other phenotypes.

Secondly, the exquisite specificity for a single receptor within a class, which is the holy grail of conventional pharmaceutical development paradigms, even when it has eventually been achieved, has not turned out to be particularly beneficial. Because of the degeneracy of the chemokine system, with many related ligands binding to the same receptor, and several receptors able to transduce the signal due to most ligands, agents which block a single receptor usually have disappointingly weak effects on the immune system, with no general anti-inflammatory properties. A good analogy is with a music amplifier: an anti-inflammatory effect would require modulation of the "volume control" but specific chemokine receptor antagonists act on the "graphic equaliser". In other words, such agents may subtly modulate the type of the inflammatory response (perhaps altering the cellular composition of the infiltrate, in terms of the leukocyte subsets present), but they do not quantitatively reduce the degree of inflammation.

As a result, even in 2009, more than 20 years after the first description of the chemokine system and its subsequent emergence as a key immunomodulatory target, no chemokine inhibitor has been approved for use as a human pharmaceutical (although several are reaching later stage clinical trials in certain indications where modulation of the type of the inflammatory response has some benefits, such as the trial of BX471, a specific antagonist of the CCR1 chemokine receptor (Horuk (2005) Mini Rev. Med. Chem. 5:791-804), in rheumatoid arthritis). Consequently, an alternative approach is needed: compounds which block the signals due to many chemokines simultaneously (agents which we have termed broad-spectrum chemokine inhibitors, or BSCIs; Grainger & Reckless (2003) Biochem. Pharmacol. 65:1027-34; Grainger et al. (2005) Mini Rev. Med. Chem. 5:825-32).

Compounds with broad-spectrum chemokine inhibitor (BSCI) activity are a new class of agents useful as anti-inflammatory medicaments to treat or prevent a wide range of diseases. In animal models of human diseases, BSCIs have been shown to be effective at reducing leukocyte accumulation, and other markers of the severity of the disease, in atherosclerosis, stroke, asthma, surgical adhesion formation, endometriosis, dermatitis, endotoxemia and rheumatoid arthritis (Reckless et al. (2001) Immunology 103:244-54; Beech et al. (2001) J. Cereb. Blood Flow Metabol. 21:683-9; Grainger & Reckless (2003) Biochem. Pharmacol. 65:1027-34; Naidu et al. (2003) Ann. Thorac. Surg. 78:1118-22; Naidu et al. (2004) J. Heart Lung Transplant. 23:128-34; Reckless et al. (2005) J. Vasc. Res. 42:492-502; Berkkanoglu et al. (2005) Hum. Reprod. 20:3047-52; Kayisli et al. (2007) Reprod. Sci. 14:825-35; Hildebrandt et al. (2009) *in the press).* BSCIs have also been shown to suppress HIV replication in vitro (an activity unrelated to their anti-inflammatory properties, resulting from the chemokine-dependency of HIV cellular entry; Grainger & Lever (2005) Retrovirology 2:23).

Despite possessing powerful anti-inflammatory activity in such a wide range of disease models, BSCIs as a class are remarkably devoid of toxicity or more minor side-effects (Schroff et al. (2005) Mini Rev Med Chem. 5:849-55). Aminolactam BSCIs, for example, have been evaluated in a wide range of acute and chronic toxicology studies by a number of routes of administration, with no-effect levels at least 1,000 fold higher than the maximum therapeutic dose. These aminolactam BSCIs also display excellent safety pharmacology profiles (with no effect on hERG tail currents, or in assays of genotoxicity; see for example WO2009/016390).

Taken together, these findings strongly suggest that BSCIs, as a class, can offer a superior trade-off between efficacy and side-effects compared with existing anti-inflammatory medications (including corticosteroids and NSAIDs). As a result, there is a need for methods to identify compositions with BSCI activity.

Unfortunately, the BSCIs described to date were identified serendipitously, and there is no straightforward method for identifying new, potentially structurally unrelated, BSCIs. The first compound with BSCI activity was a peptide, termed Peptide 3, which was selected from the sequence of MCP-1 and tested for the ability to bind to multiple chemokine receptors (Reckless & Grainger (1999) Biochem. J. 340:803-11). Although it transpired that Peptide 3 did not bind multiple chemokine receptors (but in fact binds to a single unidentified high affinity site on the cell surface; Grainger & Reckless (2003) Biochem. Pharmacol. 65:1027-34), it did nevertheless possess BSCI activity against at least five chemokines between them capable of binding to at least 10 different receptors.

From this Peptide 3 structural template, a series of different structural families of BSCIs (including peptide, peptoid and non-peptide structures) were designed, synthesised and characterised. Importantly, the structural motif responsible for the BSCI activity of the peptide was narrowed down to a WxQ tripeptide (where W represent the amino acid tryptophan, x represents any proteogenic amino acid and Q represents glutamine). This allowed the design of non-peptide WxQ mimetics, one of which (the N-acyl glutarimide NR58,4) has nanomolar activity as a BSCI (Fox et al. (2002) J. Med. Chem. 45:360-70).

Although active as a BSCI in vivo (with potent anti-inflammatory effects in models of acute inflammation, such as endotoxemia), it soon emerged that NR58,4 was not an ideal compound for pharmaceutical development. The key imide ring was susceptible to hydrolysis by an unidentified amidase in plasma, and as a result the compound was rapidly inactivated in vivo (Fox et al. (2005) J. Med. Chem. 48:867-74).

Further medicinal chemistry studies then identified a related series of substituted aminolactams that retained potency as BSCIs, but were now stable in vivo. Examples of this class of BSCIs include N-oleoyl-3-aminocaprolactam (BN83253; Fox et al. (2005) J. Med. Chem. 48:867-74). A wide range of other structurally related aminolactam BSCIs have also been described (see for example WO2005/053702, WO2006/01615, WO2006/018609, WO2006/085096; WO2006/134385, WO2006/134384 and WO2009/016390), at least some of which apparently have suitable properties for development as human pharmaceuticals.

Another family of non-peptide BSCI has also been identified, based on the structure of the key WxQ pharmacophore. The alkaloid yohimbine (an alpha2-adrenoceptor agonist derived from the root of *Rauwolfia Serpentina)* has a carbon skeleton which resembles a particular conformation of the WVQ tripeptide, and 17-amide derivatives of yohimbine are highly active as BSCIs (WO99/12968; Grainger et al. (2005) Mini Rev Med Chem. 5:825-32).

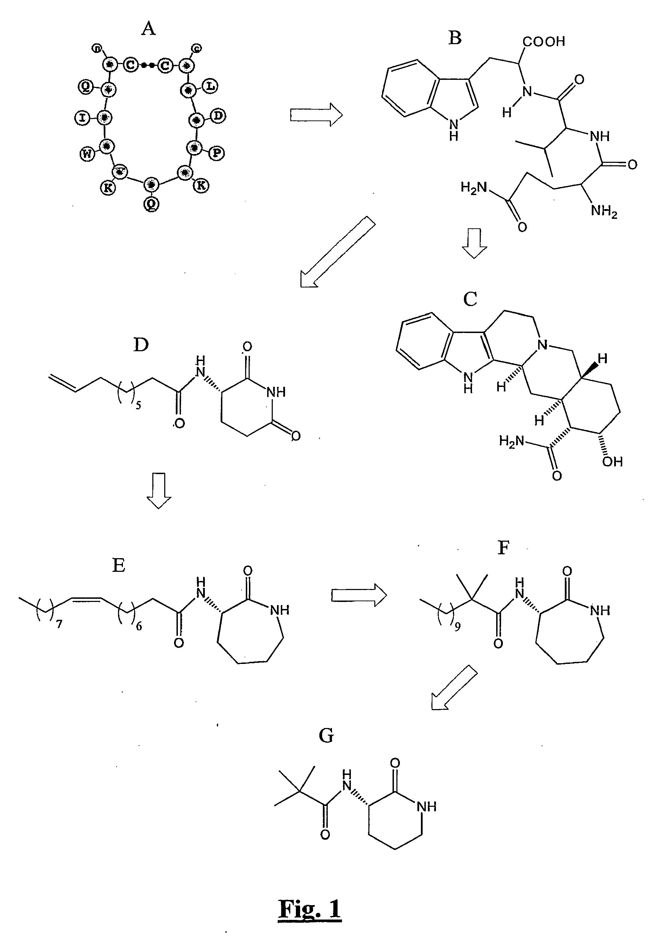

The structural inter-relationships between all the families of BSCIs described to date are illustrated in Fig. 1. It is clear from this illustration that all the families of BSCIs have been identified by the similarity of their chemical structure to each other.

No general method currently exists to identify BSCIs which are not structurally related to the known compounds. The reason for this limitation is lack of knowledge about the molecular target for BSCIs. Once it became clear that these BSCIs do not act as chemokine receptor antagonists, and do not bind to any of the chemokine receptors tested, but instead bind to a distinct (but unidentified) high affinity site on the cell surface (Grainger & Reckless (2003) Biochem. Pharmacol. 65:1027-34), it was then impossible to use conventional molecular screening tools (such as receptor binding assays) to seek new, structurally distinct families of BSCIs. While it may be possible to screen very small libraries directly for BSCI activity using functional screening tools (that is, asking whether library elements inhibit cell migration in response to several different chemokines, but not to a non-chemokine), such screening strategies rapidly become impractical due to the labour-intensive nature of the functional screening assays (see Frow et al. (2004) Med. Res. Rev. 24:276-98 for an extensive review of the technical difficulties of performing functional screening assays to identify inhibitors of cell migration). Furthermore, such functional screening can only be applied to compounds which are soluble in biologically compatible buffers and which exhibit low cellular toxicity; parameters which exclude the vast majority. (certainly more than 90%) of the compounds in a typical random library used for high throughput drug screening. In short, such an approach is not at all feasible for locating entirely new classes of BSCIs from random compound libraries.

The ability to identify new, structurally distinct BSCIs is important for the clinical development of this drug class. As existing BSCIs progress through clinical development it is not unlikely that properties will be identified which are less than optimal. To date, no BSCI compound has ever been administered in man, so the risk of hitherto unidentified side-effects or toxicities remains substantial. Worse still, a significant proportion of side-effects and toxicities which may be revealed are likely to be class effects, shared by families of compounds with related structures. Unfortunately, therefore, since for BSCIs all the families of compounds described to date were identified as a result of their structural similarity, the risk of finding a shared side-effect is heightened.

The present invention, as defined in the appended claims and below, is based in part on our description of the molecular target of the peptide and aminolactam BSCIs, which provides the first molecular screening method useful to identify structurally diverse families of BSCIs which share the mechanism of the known drug candidates, but do not necessarily contain the same structural motifs.

According to the present invention there is provided a method for the identification of a compound or agent BSCI activity comprising the following steps:
(a) firstly one or more candidate compounds or agents other than somatostatin are screened for binding to a somatostatin receptor, for example the type 2 somatostatin receptor (sstr2) such as sstr2A or sstr2B;
   then (b) compounds or agents which show binding to the somatostatin receptor are tested for BSCI activity in a functional assay,
   wherein the method excludes treatment of a human or animal body by surgery or therapy.

The invention provides a method where one or more compounds or agents are screened for interaction with the BSCI target receptor, and then in a second step those compounds or agents which interact with the target receptor are screened for the ability to inhibit leukocyte migration induced by a chemokine. Compounds or agents that inhibit chemokine-induced leukocyte migration are then tested for BSCI activity in vitro. Importantly, not all compounds or agents that are agonists at the target receptor have BSCI activity. As a result, the two steps of the invention are both necessary and sufficient to identify compounds with BSCI activity.

Since the target receptor for peptide and aminolactam BSCIs is disclosed herein (see the Examples below) as the type 2 receptor for somatostatin (sstr2), the method of the invention therefore in one aspect involves screening one or more compounds for interaction with sstr2.

As an example of the application of the method of the invention, the compound or compounds (or one or more agents) to be screened are first tested for their ability to interact with sstr2 by exposing cells expressing sstr2 to a radioligand capable of binding to sstr2 (such as labelled somatostatin) in the presence and absence of the test compounds. Where the test compounds interact with the sstr2 receptor, then the amount of somatostatin specifically bound to the receptor will be altered: Such a receptor binding assay could be performed in many different ways to achieve the same end: different ligands could be used (such as the sstr2-specific antagonist BIM23627; Tulipano et al. (2002) **143:**1218-24); different reporter systems could be used to visualise ligand binding (scintillation proximity or fluorescence polarisation are just two such examples); different cells, including cell membrane preparations or cells expressing sstr2 from a recombinant system could be used; different vehicles could be used to expose the cells to the test compounds; different buffer conditions could be used to observe the specific binding of the ligand and so forth.

It follows from the above that somatostatin *per se* may be excluded as a candidate compound or agent according to the invention.

In a second step, those compounds or agents that demonstrated interaction with the sstr2 receptor are screened for the inhibition of chemokine-induced leukocyte migration. For example, leukocytes are placed in a Transwell^{RTM} migration assay plate in the presence of a gradient of the chemokine MCP-1, and incubated at 37°C for 2 hours in the presence or absence of the test compound(s) (Frow et al. (2004) Med. Res. Rev. 24:276-98). The number of cells which have undergone migration at the end of the experiment is then determined, such as with the vital dye MTT and interpolation to a standard curve. Where the test compounds inhibit chemokine-induced migration there will be less undergoing migration in wells receiving the compound. Such a migration assay could be performed in many different ways to achieve the same end: different migration chambers could be used (multiwell format Transwell^{RTM} migration chambers, conventional Boyden chambers, in-gel migration wells, Dunn Chambers and so forth); different chemokines could be used (SDF-1α, IL-8, MIP-1α, RANTES etc); different leukocytes could be applied to the chambers (including freshly prepared peripheral blood neutrophils or lymphocytes, peritoneal macrophages or cell lines such as THP-1 or Jurkat); different detection systems can be used to quantify the number of cells migrated and so forth.

The method of the present invention could readily be applied to screen a library of compounds or agents to identify which one(s) were most likely to possess BSCI activity. Such a library could be a random library (for example, a large library of structurally diverse compounds used by many pharmaceutical companies for high throughput screening), or a directed library (for example, a library synthesised by combinatorial chemistry applied to a restricted number of precursor compounds). Certain directed libraries may be more likely to contain BSCI compounds (or, indeed, conventional sstr2 agonists), and as a result such libraries may be particularly useful when screened with the method of the present invention. For example, the G-protein Agonist Enriched Library (GAEL) library, which we have disclosed previously (see WO2006/085096), may be productively screened according to the method of the present invention since it is now clear that BSCI compounds are acting as a special class of agonist at a particular G-protein Coupled Receptor (sstr2). The use of directed libraries, such as GAEL, may reduce the number of compounds that need to be screened in order to identify a compound with candidate BSCI activity according to the criteria disclosed herein. However, the method according to the present invention is sufficiently high throughput that it can be successfully applied to random libraries, and such applications are encompassed by the invention.

Optionally, the activity of the BSCI as a "classical" somatostatin agonist may also be determined. Preferably, the agents identified according to the method of the invention are active in both types of functional assay (that is, a cell migration assay for BSCI activity and a functional assay for classical somatostatin-like activity), but are more potent (that is, they act at lower concentrations) inhibitors of chemokine-induced leukocyte migration than their potency as classical somatostatin receptor agonists.

In other words, optimal BSCIs can be identified according to the method of the invention as compounds having all of the following three properties:
(i) the compound interacts with the sstr2 receptor with an affinity of at least 10µM (that is, when the compound is present at 10µM, more than half of the sstr2 receptor present have compound bound to them);
(ii) the compound inhibits leukocyte migration induced by chemokines with a potency of at least 1µM (that is, when the compound is present at 1 µM, the extent of cell migration is inhibited by at least 50%); and
(iii) the potency of the compound in one or more assays of classical sstr2 agonist activity is lower than the potency for the inhibition of leukocyte migration.

More preferably, the compound will interact with the sstr2 receptor with an affinity of at least 1µM; more preferably, the compound will inhibit chemokine-induced leukocyte migration with a potency of at least 100nM; more preferably the potency of the compound against leukocyte migration will be more than 10-fold greater than the affinity of the interaction with sstr2 or the potency of classical sstr2 agonist activity.

Still more preferably, the compound will inhibit chemokine-induced leukocyte migration with a potency of at least 10nM; more preferably, the potency of the compound against leukocyte migration will be more than 50-fold greater than the affinity of the interaction with sstr2 or the potency of classical sstr2 agonist activity.

It is evident that the method of the invention is able to distinguish compounds with BSCI activity from conventional sstr2 agonists, such as somatostatin-14, octreotide, lanreotide or L-779,976 (Rohrer et al..(1998) Science 282:737-40). Compounds that are conventional sstr2 agonists may inhibit chemokine-induced leukocyte migration but they only do so with similar potency, or lower potency, to the affinity of their interaction with sstr2.

Similarly, conventional sstr2 agonists only exert effects on chemokine-induced leukocyte migration at similar concentrations to which they exert conventional effects through sstr2 (such as inhibition of growth hormone release or modulation of insulin secretion in the pancreas). As a result, conventional sstr2 agonists may possess anti-inflammatory activity at certain doses, but such compounds also exert the effects of somatostatin acting through sstr2 at the same dose. It should be emphasised that a key advantage of the present method is not simply to identify agents with anti-inflammatory properties but to identify a superior sub-class of agents whose anti-inflammatory properties exceed the side-effect burden. Specifically, the agents identified have greater potency as anti-inflammatory agents than as classical sstr2 agonists (a property not shared by all, or most likely even the majority, of sstr2 agonists).

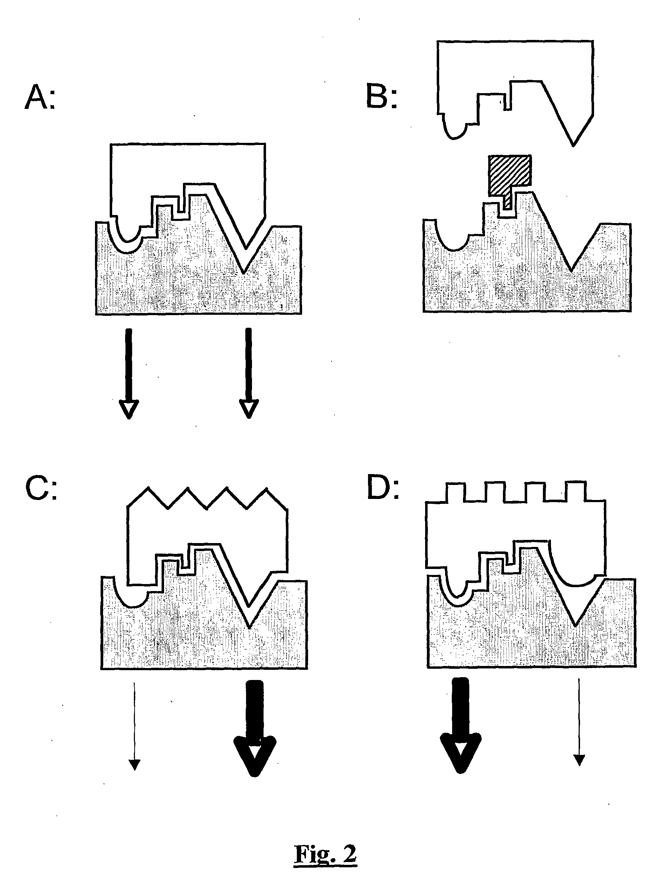

Peptide and aminolactam BSCIs are a special (and previously unidentified) subset of sstr2 agonists. Although these BSCIs and conventional sstr2 agonists share a common molecular target (the sstr2 receptor), the two groups of agents cause a different outcome on binding to the receptor. This concept has been described at length elsewhere for other receptors (Urban et al (2007) J. Pharmacol. Exp. Ther. 320:1-13), and is termed functional selectivity. A useful analogy is to consider the receptor (in this case sstr2) as a piano keyboard: many different hand positions (equivalent to different ligands) can interact with the keyboard (equivalent to productive binding), but each generates a different chord sound (equivalent to a different functional outcome from the receptor, such as different second messengers being produced). The concept of functional selectivity is illustrated in Fig. 2.

The knowledge, disclosed here, that peptide and aminolactam BSCIs are a special class of functionally selective sstr2 agonists allows compounds with BSCI activity to be identified without any pre-existing structural information (that is, without knowledge of the pharmacophores common to previously disclosed families of BSCI compounds).

Such compounds may be identified according to the method of the invention, for example as follows. In a first step, compounds are screened for interaction with the sstr2 receptor. Next, BSCIs are distinguished from conventional sstr2 agonists by the ability to inhibit chemokine-induced leukocyte migration with higher potency than its sstr2 agonist activity against conventional targets (such as suppression or growth hormone release). In an optional fourth step, structural motifs may be identified which define new classes of BSCIs structurally unrelated to those previously disclosed. Typically, such a step would involve comparing structural elements among the compounds newly identified as BSCIs according to the method of the present invention, such that recurring structural elements are likely to correspond to motifs required to confer BSCI activity. The importance of such motifs can then be verified experimentally, for example by synthesising a range of molecules with related structures, to demonstrate that the presence of the motif is associated with the presence of BSCI activity. As a result, the method of the invention may be used to define whole classes of BSCI compounds (members of each class being related in respect of their structure to one another), even if only a single member of the class were originally identified through screening for interactions with sstr2.

Thus it is envisaged that BSCI compounds identified by the method according to the present invention would act as the structural lead for the synthesis and characterisation of a family of novel, structurally related BSCIs. As a result, while such structural derivatives of the initial hit identified by the method of the invention, are not directly identified through the application of the method of the invention, nevertheless the key step in their identification depended on the method of the invention, and consequently the indirect identification of a family of related BSCIs in this way is envisaged according to this aspect of the invention.

Where a library is to be screened according to the method of the present invention, the elements of the library may be individual compounds (or at least substantially pure compounds, where the target compound represents at least 25% of the material present, with no other compound present at higher levels), or else the elements of the library may be mixtures of compounds. Where the elements are mixtures of compounds, the components of the mixture may be known or predicted (as in many combinatorial libraries) or unknown (for example, in natural product isolates).

In the event that some or all of the library elements being screened according to the method of the invention are mixtures (whether known or unknown in composition), it is envisaged that after the first step of the method of the invention, the particular compound from the mixture which is responsible for the interaction with sstr2 will be isolated and identified prior to application of the second step (assessment of the effect of the compound on chemokine-induced leukocyte migration). If such an isolation step were not included in the method, then the method could not distinguish the case where a single compound in the mixture had BSCI activity (as desired) from the case where one compound in the mixture had conventional sstr2 agonist activity and a second unrelated compound inhibited leukocyte migration by a mechanism unrelated to BSCI activity.

For example, if the method of the invention were applied to a library element which consisted of a mixture of somatostatin-14 (a conventional sstr2 agonist but not a BSCI) and sodium azide (a cellular toxin which blocks cell migration but is not a BSCI), this mixture would show a strong interaction with sstr2, and (depending on the relative concentration of azide present) inhibit chemokine-induced leukocyte migration with a higher potency than predicted from the affinity at sstr2. Such a mixture would be incorrectly identified as a BSCI. However, if as described here, the component of the mixture responsible for the interaction with sstr2 (in the case of this example, the somatostatin) was isolated from the mixture prior to application of the second step to assay effects on chemokine-induced migration, then the method would correctly identify the mixture as lacking BSCI activity.

While in principle, the steps of the method of the invention could be applied in any order (for example, it would be possible in principle to determine the potency of the compound(s) under study as inhibitors of chemokine-induced migration and subsequently determine the affinity of the interaction (if any) of the compound with sstr2) such a method lacks the benefits of the preferred aspect of the present invention. In these circumstances, random libraries could not be screened using the functional assay (due to the presence of toxic and insoluble compounds, as well as due to the resource implications of such a screen). In the present invention as preferred, therefore, screening for interaction with sstr2 will be performed as the first step, followed by a functional assay for inhibition of chemokine-induced leukocyte migration. This order of the steps reflects the optimal use of available resources, since the screen to assess interaction with sstr2 (an example of a molecular screen) requires considerably less resources per compound screened than the determination of activity against chemokine-induced leukocyte migration (an example of a functional screen).

Application of the method of the invention is envisaged to include virtual screening as well as practical or physical screening. In other words, some (or all) of the information needed to identify a compound as a candidate BSCI according to the method of this invention may already be available. For example, a library of compounds may already have been screened for interaction with sstr2 prior to the present disclosure, and the results be stored (for instance, on a computer hard drive). As a result, a subset of compounds from the library may.be selected and then tested for potency as inhibitors of the chemokine-induced migration in leukocytes and identified, according to the method of the invention, as candidate BSCIs. It is evident that such a process still represents an application of the method disclosed herein, and depends upon the disclosed criteria in order to identify candidate BSCIs. Similarly, in the event that, for a series of compounds, the affinity of the interaction of each with sstr2 was already known, and separately the potency of the compounds for the inhibition of chemokine-induced leukocyte recruitment was already known, then the combination of the existing information to identify which of the compounds were candidate BSCIs would still represent an application of the method of the invention, even if no de novo experimentation was required at all.

Particularly where one or more steps of the invention do not require de novo experimentation, the steps may be performed in any order (i.e. not necessarily in the preferred order noted herein).

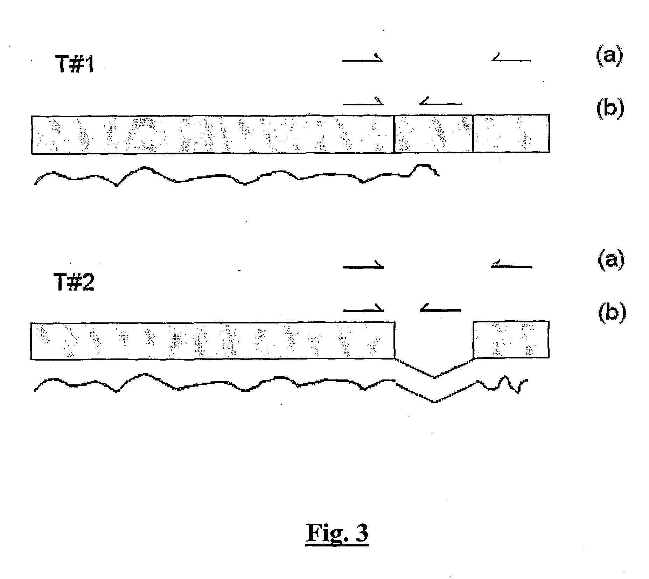

The sstr2 receptor, encoded by the sstr2 gene, exists in at least two different variants, resulting from alternative splicing (Olias et al. (2004) J. Neurochem. 89:1057-91 and the references therein) designated sstr2A and sstr2B (Fig. 3). The spliced mRNA encoding sstr2A has an additional exon, which is skipped in the sstr2B transcript. This exon includes a stop codon, and as a result the sstr2A and sstr2B proteins differ in amino acid sequence only in the short C-terminal cytoplasmic tail. It is unclear what, if any, functional significance this alternative splicing may have, but different cell types express different ratios of the two splice variants (at least at the mRNA level). Since the two variants are identical in the extracellular ligand binding loops, none of the pharmacological agents used to study sstr2 functionally distinguish the two (so sstr2-specific antagonists prevent ligand binding to both variant forms). As a result, either or both of the variant forms may be used for the methods described herein.

All cell lines which have been tested to date that are sensitive to BSCIs express either sstr2A only or both sstr2A and sstr2B. Furthermore, we know that ectopic over-expression of sstr2A confers sensitivity to BSCIs in resistant cell lines (see Example 5 below). As a result, a preferred embodiment of the present invention employs data from cell lines expressing sstr2A (either alone or in combination with sstr2B). Such cell lines may naturally express sstr2, or may have been engineered (by methods well known in the art) to express sstr2.

Expression of somatostatin receptors on leukocytes is already known (for example, see Hiruma et al. (1990) Immunology 71:480-5; Tsutsumi et al. (1997) Cell. Immunol. 181:41-9; ten Bokum et al. (1999) J. Endocrinol. 161:167-75 and the references therein; Elliot et al. (1999) Eur. J. Immunol. 29:2454-63; Talme et al. (2001) Clin. Exp. Immunol. 125:71-9; Dalm et al. (2003) Am. J. Physiol. Endocrinol. Metab. 285:E344-53; Lichtenauer-Kaligis et al. (2004) Eur. J. Endocrinol. 150:565-77). Furthermore, there have been descriptions of anti-inflammatory activities attributable to somatostatin and other somatostatin receptor ligands (for example, see Paran & Paran (2003) Curr. Opin. Invetig. Drugs 4:578-82; Sener et al. (2005) Peptides 26:493-9; Gonzales-Rey et al. (2007) Ann. Rheum. Dis. 66:582-8 and the references therein; Armani et al. (2007) J. Leukoc. Biol. 81:845-55; Cury et al. (2008) Dig. Dis. Sci. 53:2516-20). As a result, the novelty and inventive step of the present invention does not lie in the discovery that ligands acting through somatostatin receptors can have clinically useful anti-inflammatory effects (a discovery which would teach an invention with only a single step, namely to screen for interactions with sstr2 to identify potentially useful anti-inflammatory agents). Instead, the invention relates to the unexpected finding that some (and in all likelihood a very small proportion) of all agonists at the sstr2 receptor have anti-inflammatory activity *substantially in place of* classical somatostatin-like activity (such as suppression of Growth Hormone secretion).

Functional selectivity of this kind (where different ligands bind to, and activate, the same receptor but elicit markedly different functional outcomes) is known in the pharmacology literature (see Urban et al (2007) J. Pharmacol. Exp. Ther. 320:1-13 for a review), but robust examples are few. As a result, it would be impossible to guess whether functionally selective ligands might exist for any given receptor, or for any given pair of functions. Prior to our disclosure, therefore, one skilled in the art would have reasonably concluded that any anti-inflammatory activity associated with sstr2 activation would necessarily have been accompanied by classical somatostatin-like responses, limiting the usefulness of sstr2 as a target for the discovery of novel pharmaceuticals with anti-inflammatory activity. In our disclosure, we show unequivocally that functional selectivity does occur at sstr2, and moreover that it is possible to exert substantial anti-inflammatory activity with little or no classical somatostatin-like activity. This finding had not been predicted, nor could it reasonably be assumed to be possible, let alone likely, even by those most skilled in the art (including the present inventors) on the available information prior to our disclosure. The present invention provides a method for the unambiguous identification of agents which display functional selectivity, favouring BSCI activity over classical somatostatin-like activity, at sstr2. It is not necessary to understand any molecular details of how this functional selectivity is achieved, nor the structural characteristics among ligands that define the desired outcome, in order to usefully practice the present invention. The information provided in the present disclosure is both necessary and sufficient to teach one skilled in the art how to select potentially useful functionally selective agents from among a pool of otherwise equally promising candidates. Nevertheless, it may be useful to further illustrate the extent to which the present findings are unexpected, and to consider how functional selectivity must be achieved.

When a ligand binds to a receptor, there are multiple non-covalent bonding interactions between the ligand molecule and the receptor protein (including, but not limited to, Van der Waal's forces, hydrogen bonding, dipole interactions and salt bridges; see Fig. 2). These interactions are responsible for both the affinity of the interaction between ligand and receptor (and hence the selectivity of binding to target receptor(s) as opposed to other proteins) and also any signalling response to ligand binding. For the natural ligand, these interactions result in changes in the conformation of the receptor protein (the lowest energy conformation of the receptor:ligand complex is markedly different from that of the receptor and the ligand when separated). This conformational change transduces the signal from ligand binding to the outside of the cell into intracellular signalling cascades. The transduction may be direct (as in the case of receptors with endogenous kinase activity) or indirect (as in the case of G-protein coupled receptors, such as sstr2) where the conformational change promotes the association of one or more further proteins that in turn transduce the signal. Any agent that binds to the receptor and replicates the conformational change in the receptor seen with the natural ligand will be an agonist at the receptor (Fig. 2A). Any agent that binds to the receptor without inducing those changes will be an antagonist (assuming it blocks the binding of the natural ligand; Fig. 2B). However, under unusual circumstances a third possibility arises: the agent may bind and induce a very different set of conformational changes that cause a qualitative, rather than quantitative, change in the nature of the signal which is subsequently transduced (Fig. 2C & D). It is this third, unusual, scenario which underpins the pharmacological phenomenon of functional selectivity (and, to some extent, of superagonists and inverse agonists).

This molecular scenario may be better understood by reference to a musical analogy. In this model, a piano keyboard is the analog of the receptor, and the hand of the pianist is the ligand. Under normal circumstances the interaction of the hand (in a particular conformation) strikes a particular chord. This "signal" represents the agonist response at the receptor. Closing the piano lid prevents the interaction of the ligand and the receptor without inducing any conformational change in the key positions, and hence no chord (agonist signal) is heard. The piano lid is an analog of an antagonist. Replacing the pianist with a child uninstructed in keyboard playing (the analog of a randomly selected compound from a library) will result in almost every case in an uninterpretable cacophony, rather than in a musical chord. Very occasionally, however, a child genius may be encountered who plays a harmonious chord, very different from the pianist. The same receptor has been made to respond with functional selectivity: different ligands have elicited qualitatively different responses from the same receptor.

It will be evident from the foregoing that there presently exists no method to predict the functional outcomes from different ligands binding to the same receptor. Indeed, it is not even possible to predict which signals are possible for a given receptor (in the way that a violin sound could never be generated from a piano keyboard however you depressed the keys). Fortuitous discovery, followed by empirical screening based on that disclosed information, is a way to discover agents with desirable functional selectivity, such as the BSCIs described herein.

It is evident that the present invention consists of two or more steps, each of which in isolation was a process known in the art. For example, the first step has been used on a number of occasions for the purpose of locating agents that interact with somatostatin receptors either to replicate the known effects of the natural ligand or to block the known effects of the natural ligand. Similarly, we and others have disclosed the use of a functional assay of chemokine-induced leukocyte migration for the purpose of characterising agents for BSCI activity. However, prior to the present disclosure there was no possible rationale to link these two processes, and to perform them in series, so as to achieve an entirely different outcome (the ability to screen for compounds with BSCI activity in a random library, which cannot be achieved using either step in isolation since most somatostatin receptor interacting agents are not BSCIs and most elements in a random library are not compatible with the functional assay for BSCI activity). However, knowledge that peptide and aminolactam BSCIs are a special subclass of agonists at sstr2, acting by functional selectivity, which is disclosed for the first time in the present document, enables the present invention by combining these two unrelated processes into a novel procedure which has unexpected utility. A good analogy of this inventive process is the composition of a new peptide: peptides are (almost all) composed of the same amino acid building blocks, linked in the same ways. However, there are so many possible combinations that the identification of a particular combination (a new peptide sequence) that has a particular function is considered inventive. Similarly, there are so many possible receptors at which BSCI could have acted (even if the search were restricted to those known to be able to mediate anti-inflammatory activity in vivo) that it was impractical to try and combine molecular screens at each of them with functional screens for BSCI activity. The present invention overcomes that impractical search for useful combinations, by providing a method for identifying BSCIs from a random library which is unexpectedly and particularly superior to many other combinations that were *ab initio* equally plausible.

The methods described herein could be applied equally to homologs of sstr2 in other species, including, but not limited to mouse, rat, dog and primates, as well as in non-mammalian species such as birds. If a non-human homolog of sstr2 is used for the purpose of identifying compounds according to the present invention which are intended for use in man (for example as anti-inflammatory medicaments) then an additional step may be required in which the agent identified using the non-human receptor is subsequently checked for functional selectivity at the human receptor. Because of the substantial sequence and functional homology among human sstr2 and its homologs in other species, it is expected that a sufficient majority of those agents identified as BSCIs using the methods of the present invention applied to non-human sstr2 will also be BSCIs acting through human sstr2 that use of non-human sstr2 can be considered interchangeable with the use of the human receptor. Preferably, however, if BSCI activity in a human system is the desired outcome of the screening exercise, then the methods of the invention should be applied to human sstr2. Of course, if the desired outcome is BSCI activity in a non-human system (for example, as a veterinary medicament) then it would be most appropriate to use the sstr2 analog from one or more of the target species.

It is envisaged that the method of the invention could be applied using incomplete fragments of the receptor, provided that the fragment of the receptor retains the ability to bind to somatostatin or another ligand of the full length receptor. Typically, such functional fragments will contain the majority of the protein sequence, but parts that are not involved in ligand binding may be deleted (for example, to improve expression or stability) without affecting the application of the present invention.

It will be evident that binding of the test agent to the receptor (sstr2) could be determined indirectly (through competition for binding with another ligand for the receptor, which had previously been labelled) or directly (where the test agent itself bears the label). The scope of the present invention is in no way intended to be limited by the methodology employed to detect the interaction of the test agent with the receptor.

It will be evident that the functional assay used to detect the BSCI activity of the test agents could be performed in vitro (for example using a Transwell^{RTM} migration assay) or in vivo (for example using intraperitoneal injection of a chemokine, followed by assessment of leukocyte recruitment in response). Furthermore, it is possible to use surrogate markers of leukocyte recruitment (both in vitro and in vivo) for the assessment of BSCI activity. For example, leukocytes (particularly when activated) are known to express the cytokine TNF-alpha. As a result, measuring levels of TNF-alpha in vitro or in vivo represents an alternative method for assessing leukocyte recruitment, and therefore of estimating BSCI activity. The scope of the present invention is in no way intended to be limited by the methodology employed to detect BSCI activity associated with a particular test compound.

It will be evident that the functional assay used to detect classical sstr2 agonist activity of the test agents could be performed in vitro (for example by measuring GH release from pituitary cells) or in vivo (for example by measuring GH levels in serum). Furthermore, it is possible to use surrogate markers to estimate classical sstr2 agonist activity. For example, GH regulates the level of insulin-like growth factor I (IGF-I) such that IGF-I levels may be measured as an indicator of GH levels, and therefore as an estimate of classical sstr2 agonist activity. The scope of the present invention is in no way intended to be limited by the methodology employed to detect classical sstr2 agonist activity associated with a particular test compound.

Optionally, an additional step may be added to the method of the invention, to allow the identification of novel structural classes of "BSCI-type" sstr2 agonists, or "somatotaxins" based on the presence of one or more structural features of the compounds selected during the prior steps of the method of the invention. Identifying such structural motifs will allow, through application of the method of the invention, a much wider array of "BSCI-type" sstr2 agonists or "somatotaxins" than simply those compounds whose properties were probed either experimentally or theoretically in order to identify leads from among a library (whether random or composed of selected candidates). For example, following screening of a random library for binding to sstr2 (the first step of the present invention), followed by determination of the inhibition of chemokine-induced leukocyte migration (a second step) and optionally determination of the potency against "classical" sstr2 targets, several new compounds may be selected according to the method of the invention. Two or more of these selected compounds may share a common structural motif, such as the presence of 3-substituted cis-decalin ring system. In the optional additional step, therefore, it is evident that one might suppose the presence of a 3-substituted cis-decalin ring system may confer the desirable properties on the compounds, and further 3-substituted cis-decalin containing molecules could be synthesised with the reasonable expectation that some, at least, of them would have desirable properties. In such circumstances, the definition of the new structural motifs associated with "BSCI-type" sstr2 agonist activity, or "somatotaxin" activity would constitute an application of the method of the present invention.

Thus, the method of the invention may comprise a minimum of two steps (determination of interaction with sstr2, followed by a functional assay for BSCI activity). Optionally, a third step may be added (a functional assay for "classical" sstr2 agonist activity). The advantage of this third step is that it identifies a sub-group within the sstr2-directed BSCIs which have greater potency for the inhibition of leukocyte migration than they have potency as "classical" sstr2 agonists (that is, it identifies the "somatotaxin" subclass). This additional restriction likely identifies those compounds with the greatest potential for development as anti-inflammatory medicaments, with maximum efficacy and fewer side-effects due to unwanted sstr2-mediated effects, such as those on pituitary hormones or glucose handling. However, depending on the particular application, the resource implication of adding this optional third step may exceed the benefit associated with the improved definition of the functional profile of the compounds under investigation. Optionally, a final step may be added (defining a structural motif associated with BSCI or somatotaxin activity), whether or not the optional third step had been applied previously. An advantage of this final step is that it allows compounds with desirable properties to be selected through structural homology rather assay-based screening. The methods for such "hit to lead" optimisation programmes are well known in the art, and facilitate the application of the method of the invention to the discovery of new agents suitable for use as human pharmaceuticals. However, depending on the particular application, the resource implication of performing this optional final step may exceed the benefit associated with the expanded range of compounds identified.

As used herein, the terms "compound" and " agent" are generally deemed to be synonymous and thus interchangeable unless suggested other wise by context.

The term "broad-spectrum chemokine inhibitor" or "BSCI" refers to a compound or agent which can inhibit cell migration in response to a wide range of chemokines that normally signal through different receptors to induce migration, while having little or no impact on cell migration induced by non-chemokine chemoattractants. It is evident that this is an operational definition, and different compounds or agents may be BSCIs as a result of entirely different molecular mechanisms. The present invention, however, is concerned only with the identification of BSCIs which act through signalling at sstr2, or related receptors, identified herein as an important and extensively studied sub-category of BSCIs.

The term "BSCI type sstr2 agonist activity" refers to the effect of certain sstr2 agonists on chemokine-induced cell migration in vitro or in vivo, as well as related immunological changes in vivo, such as re-balancing of T helper (CD4+) cell cytokine secretion profiles. Agents with "BSCI type sstr2 agonist activity" are also termed "somatotaxins" herein, and "BSCI type sstr2 activity" is therefore also termed "somatotaxin activity".

The term "classical sstr2 agonist activity" refers to the constellation of cellular responses which are well known to occur, possibly in different cell types, when cells expressing sstr2 at sufficient levels are exposed to any of the known sstr2 agonist compounds, including the natural ligand somatostatin. Examples of classical sstr2 agonist activity are the suppression of GH release by cells of the pituitary gland, and suppression of insulin release by cells of the pancreas (Olias et al. (2004) J. Neurochem. 89:1057). However, a wide range of other effects of sstr2 agonists acting on various cells have also been described, which (with the exception of the "BSCI type sstr2 agonist activities" listed above) also comprise classical sstr2 agonist activity (Olias et al. (2004) J. Neurochem. 89:1057). It evident that since classical sstr2 agonist activities are defined as all those known activities of sstr2 agonists excepting the BSCI type sstr2 agonist activities, then all known activities of sstr2 agonists are captured by the present definitions.

The term "sstr2" refers to the type 2 somatostatin receptor, in humans encoded by the *sstr2* gene (HGNC ID: 11331; full gene sequence shown in SEQ ID NO: 1) on human chromosome 17q24 and encoding the amino acid sequence set forth in SEQ ID NO: 2 in the Sequence Listing below.

Unless otherwise qualified, the term sstr2 is intended to encompass the products of any alternatively spliced or otherwise differentially processed mRNA derived from the transcription of the gene locus shown in SEQ ID NO: 1. For the avoidance of doubt, the term sstr2 encompasses either or both of the sstr2a and sstr2b proteins derived from alternative splicing of the sstr2 mRNA at the single intron located in the protein coding frame. Analogs and homologs in other species are also encompassed by the term, unless to do so would be nonsense in a particular context.

The term "somatostatin receptor" refers to any receptor (whether presently known or not) to which somatostatin binds with high affinity (typically 1µM or less). For the avoidance of doubt this includes sstr1, sstr2, sstr3, sstr4 and sstr5. Analogs and homologs in other species are also encompassed by the term, unless to do so would be nonsense in a particular context.

The term "somatostatin receptor" additionally may encompass a polypeptide having an amino acid sequence which has at least 50% identity, for example at least 55%, 60%, 65%, 70%, 75%, 80%, 85%, 90%, 95% identity, or at least 96%, 97%, 98% or even 99% identity with the amino acid sequence of human sstr2 (as provided above.) The polypeptide preferably has somatostatin receptor activity as defined above.

Sequence identity between amino acid sequences can be determined by comparing an alignment of the sequences. When an equivalent position in the compared sequences is occupied by the same amino acid, then the molecules are identical at that position. Scoring an alignment as a percentage of identity is a function of the number of identical amino acids or bases at positions shared by the compared sequences. When comparing sequences, optimal alignments may require gaps to be introduced into one or more of the sequences to take into consideration possible insertions and deletions in the sequences. Sequence comparison methods may employ gap penalties so that, for the same number of identical molecules in sequences being compared, a sequence alignment with as few gaps as possible, reflecting higher relatedness between the two compared sequences, will achieve a higher score than one with many gaps. Calculation of maximum percent identity involves the production of an optimal alignment, taking into consideration gap penalties.

Suitable computer programs for carrying out sequence comparisons are widely available in the commercial and public sector. Examples include MatGat (Campanella et al., 2003, BMC Bioinformatics 4: 29; program available from http://bitincka.com/ledion/matgat), Gap (Needleman & Wunsch, 1970, J. Mol. Biol. 48: 443-453), FASTA (Altschul et al., 1990, J. Mol. Biol. 215: 403-410; program available from http://www.ebi.ac.uk/fasta), Clustal W 2.0 and X 2.0 (Larkin et al., 2007, Bioinformatics 23: 2947-2948; program available from http://www.ebi.ac.uk/tools/clustalw2) and EMBOSS Pairwise Alignment Algorithms (Needleman & Wunsch, 1970, supra; Kruskal, 1983, In: Time warps, string edits and macromolecules: the theory and practice of sequence comparison, Sankoff & Kruskal (eds), pp 1-44, Addison Wesley; programs available from http://www.ebi.ac.uk/tools/emboss/align). All programs may be run using default parameters.

For example, sequence comparisons may be undertaken using the "needle" method of the EMBOSS Pairwise Alignment Algorithms, which determines an optimum alignment (including gaps) of two sequences when considered over their entire length and provides a percentage identity score. Default parameters for amino acid sequence comparisons ("Protein Molecule" option) may be Gap Extend penalty: 0.5, Gap Open penalty: 10.0, Matrix: Blosum 62. Default parameters for nucleotide sequence comparisons ("DNA Molecule" option) may be Gap Extend penalty: 0.5, Gap Open penalty: 10.0, Matrix: DNAfull.

The term "somatostatin" refers to any of the products of the gene encoding somatostatin, in human encoded by the *sst* gene (HGNC ID: 11329) on chromosome 3q28, including fragments generated by regulated proteolysis of the gene product. Unless otherwise qualified, the term somatostatin (or sst) is intended to encompass any of the fragments generated by proteolysis to which a biological function has been assigned, including (but not limited to) sst-14 and sst-28. Analogs and homologs in other species are also encompassed by the term, unless to do so would be nonsense in a particular context.

The term "leukocyte" refers to any or all of white blood cell populations found in blood, as well as the precursors of these cells in bone marrow or secondary lymphoid organs such as spleen, and the progeny of these cells found in tissues, such as osteoclasts, Kupffer cells, microglia, mast cells, dendritic cells and so forth. Examples of leukocytes include monocytes, lymphocytes (both B lymphocytes and T lymphocytes, including killer T cells, helper T cells, regulatory T cells and Natural Killer cells) and granulocytes (neutrophils, basophils and eosinophils).

The term "potency" is used to describe the concentration of a particular substance that is required to have a particular effect. Typically potencies are quoted as the concentration of the compound required to inhibit the end-point under study by 50% (termed the ED50 or IC50). Hence, an ED50 or IC50 at a lower concentration represents a compound with a higher potency. Note that potency is entirely distinct from "power" (see below), which represents the maximal effect of the compound on the end-point under study at any concentration tested. Compounds may have high potency but low power (such as somatostatin acting on chemokine-induced migration with an ED50 of 0.05nM), but only inhibiting migration by 40% even at very high concentrations) or low potency and high power (such as an agent with an ED50 of 10µM but which inhibits the end-point by 100% at concentrations above 100µM).

The term "power", when applied.to a pharmacology assay, is used to describe the maximal effect of a particular substance on the end-point under study that can be obtained by selecting the optimal concentration of that agent in that assay. Note that the optimal concentration is typically the highest concentration tested, although in some instances (for example, where a bell-shaped dose response is commonly observed, such as in the chemotaxis-response to chemokines) a lower concentration may have the maximal effect on the end-point under study. Note that power is unrelated to potency, and a compound with low potency may still have high power and vice versa.

The term "about" refers to an interval around the considered value. As used in this patent application, "about X" means an interval from X minus 10% of X to X plus 10% of X, and preferably an interval from X minus 5% of X to X plus 5% of X.

The use of a numerical range in this description is intended unambiguously to include within the scope of the invention all individual integers within the range and all the combinations of upper and lower limit numbers within the broadest scope of the given range.

As used herein, the term "comprising" is to be read as meaning both comprising and consisting of. Consequently, where the invention relates to a "pharmaceutical composition comprising as active ingredient" a compound, this terminology is intended to cover both compositions in which other active ingredients may be present and also compositions which consist only of one active ingredient as defined.

The following abbreviations, where used, are intended to refer to the 20 naturally occurring proteogenic amino acids, whether isolated or as part of a polypeptide or other amid-containing compound as appropriate according to the context:

| **ONE-LETTER CODE** | **3-LETTER SYMBOL** | **MEANING** |
|---|---|---|
| A | Ala | Alanine |
| C | Cys | Cysteine |
| D | Asp | Aspartic Acid |
| E | Glu | Glutamic Acid |
| F | Phe | Phenylalanine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valise |
| W | Trp | Tryptophah |
| Y | Tyr | Tyrosine |

Unless otherwise defined, all the technical and scientific terms used here have the same meaning as that usually understood by an ordinary specialist in the field to which this invention belongs.

Particular non-limiting examples and further features of the present invention will now be described with reference to the following drawings, in which:
Fig. 1 shows the structures of the key BSCIs described herein, and illustrates the interrelationship (and common structural motifs) shared by all of them;
Fig. 2 is a diagram illustrating the concept of functional selectivity at a single receptor (grey shape);
Fig. 3 is a diagram illustrating two known alternative transcripts (T#1 and T#2) derived from the *sstr2* locus, as a result of alternative splicing of the single intron (darker box) in the protein coding frame;
Fig. 4 is a graph showing the impact of increasing concentrations of the sstr2 antagonist BIM 23627 ("I") on the dose-response of the BSCI BN83253 (x-axis, in nM) on MCP-1 induced THP-1 cell migration (y-axis, % inhibition);
Fig. 5 is a graph showing the impact of increasing concentrations of the sstr2 anatagonist BIM 23627 ("I") on the dose-response of the BSCI (S)-3-(2' ,2'-dimethylpropionoylamino)-tetrahydropyridin-2-one ("valerolactam"; x-axis, in nM) on MCP-1 induced THP-1 cell migration (y-axis, % inhibition);
Fig. 6 is a graph showing the impact of co-administering the sstr2 antagonist BIM 23627 with the BSCI BN83250 (x-axis, in mg/kg dose) on TNF-alpha production (y-axis, % inhibition) in response to single intraperitoneal challenge with bacterial endotoxin;
Fig. 7 is a graph showing the impact of co-administering the sstr2 antagonist BIM 23627 with the BSCI (S)-3-(2'2'-dimethylpropionoylamino)-tetrahudropyridin-2-one ("valerolactam"; x-axis, in mg/kg dose) on TNF-alpha production (y-axis, % inhibition) in response to single intraperitoneal challenge with bacterial endotoxin.;
Fig. 8 is a graph showing the impact of co-administering the sstr2 antagonist BIM 23627 with the synthetic corticosteroid dexamethasone (x-axis, in mg/kg dose) on TNF-alpha production (y-axis, % inhibition) in response to single intraperitoneal challenge with bacterial endotoxin;
Fig. 9 shows an agarose gel stained with ethidium bromide and viewed under UV transillumination, to reveal amplicons from PCR reactions with (+) and without (-) reverse transcription ("RT") from mRNA samples prepared from THP-1 cells (left panel) and Panc-1 cells (right panel), used as a positive control;
Fig. 10 provides a schema for the synthesis of the labelled analog of BN83250, 4',5'-³[H]-(S)-3-(2',2'-dimethyldodecanoyl)amino-caprolactam(4,5-³[H]-BN83250). The compound (S,E)-3-(2',2'-dimethyldodec-4'-enoyl)amino-caprolactam (top) is reacted with tritium gas in the presence of Pd catalyst to form 4,5-³[H]-BN83250 (bottom);
Fig. 11 is a graph showing the specific binding (y-axis, in cpm) of 4,5-³[H]-BN83250 (x-axis, in nM) to THP-1 cells. Values are the mean (± SD) of triplicate determinations at each BN83250 concentration, with non-specific binding (in the presence of a large excess of unlabelled BN83250) subtracted. The central line is the best-fit using a simple binding model, and the outer lines represent the 95% confidence limits;
Fig. 12 is a graph and histogram showing the effect of unlabelled (S)-3-(2'2'-dimethylpropionoylamino)-tetrahydropyridin-2-one ("Valerolactam") at various concentrations (x-axis values, in nM) on the binding of 10nM 4,5-[³H]-BN83250 to THP-1 cells (y-axis, in bound label [cpm]);
Fig. 13 shows a plasmid map of pcDNA3.1+ (Invitrogen), with the 1,100bp cDNA encoding human sstr2A (shown as "sstr2a") cloned between the EcoRI (5') and-XhoI (3') sites in the polylinker;

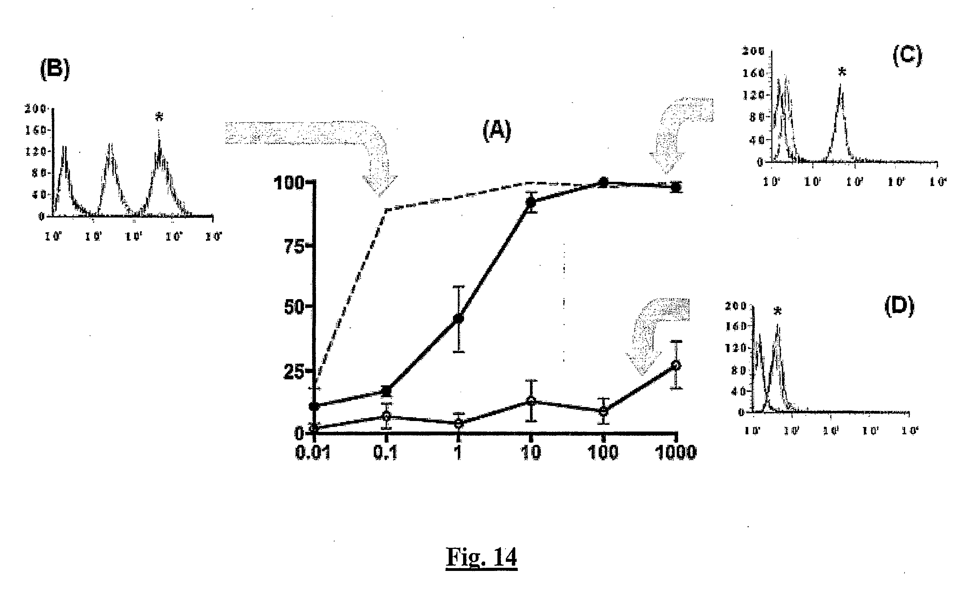

Fig. 14 compares the impact of a BSCI (BN83250) on the SDF-1α-induced migration of parental Jurkat T cells (open circles, solid line), Jurkat D3B cells expressing sstr2 (filled circles, solid line) and THP-1 cells (dotted line). In (A), the concentration of BN83250 is shown on the x-axis (in nM) while % inhibition is shown on the y-axis. Frequency histograms showing the distribution of staining for each cell line are shown in (B) for THP-1 cells (* = 375 median units of specific staining), (C) for Jurkat D3B cells(* = 40 median units of specific staining) and (C) for Jurkat T cells (* = 1 median unit of specific staining). In each frequency histogram, the x-axis represents F11-H and the y-axis count. The black line in frequency histograms (B) to (D) represents staining with PBS only; the light grey line represents the no primary antibody control, and the mid grey line represents the full staining reaction;
Fig. 15 is a graph showing the effect of various concentrations of somatostatin 14 (x-axis, in nM) on MCP-1 induced migration (y-axis, in % inhibition) of THP-1 cells. The dotted line represents complete inhibition, which is achieved with BSCIs;
Fig. 16 is a graph plotting the potency for inhibition of chemokine-induced migration (y-axis; as ED50 versus chemokine-induced migration, in nM) against the apparent affinity for sstr2 binding (x-axis; "Ka" at sstr2, in nM) for a wide range of both "classical" sstr2 agonists (side A) and "BSCI-type" sstr2 agonist (or "somatotaxins"; side B);
Fig. 17 shows the structures of a range of novel beta-methyltrytophan-based sstr2 agonists (L-799,976 and Compounds A-F), designed as candidate BSCIs for testing according to the method of the invention;
Fig. 18 is a Venn Diagram illustrating the concept which underlies the present invention, as explained below;
Fig. 19 is a graph showing inhibition of the chemokine MCP-1 induced THP-1 cell migration (y-axis, given as percentage values) by Compound P (x-axis, in nM) according to one embodiment of the invention;
Fig. 20 is a graph showing inhibition of growth hormone (GH) release (y-axis, given as percentage values) by Compound P (x-axis, in nM); and
Fig. 21 is a histogram comparing the level of C-reactive protein (CRP; y-axis, in ng/ml) as a surrogate marker for inflammation in serum samples from patients treated with Medrol^{RTM} (A) or Compound P (B). The columns show levels of CRP prior to dosing (a) and on the last day of dosing (b). "★" denotes p=0.02.

Certain figures are described in further detail below.

Fig. 1 shows NR58-3.14.3 ("A") which is a cyclic retroinverso analog of a sequence derived from Peptide 3 (not shown). The tripeptide motif WxQ was identified as the key pharmacophore, and tripeptides such as WVQ ("B") have BSCI activity (albeit at low potency). Both Yohimban-16-amide ("C") and NR58,4 ("D") were identified as structural analogs of the WxQ tripeptide. Lactams, such as BN83253 ("E"), were then identified as stable analogs of imides such as NR58,4. The introduction of 2,2-disubstitution, as in BN83250 ("F"), was later found to increase potency. Valerolactams, such as (S)-3-(2'2'-dimethylpropionoylamino)-tetrahydropyridin-2-one ("G"), have similar activity to caprolactams.

In Fig. 2, panel A shows the natural ligand interacts with the receptor and generates multiple responses (arrows), which can be interpreted as either intracellular signals or functional consequences. In panel B: an antagonist binds to the receptor and prevents the natural ligand from binding, but does not engage with the parts of the receptor necessary to induce active signalling. In panel C: a functionally selective agonist binds to the receptor in such a way as to preferentially activate one response (thick arrow) over the other (thin arrow). In panel D: a different agonist with the opposite functional selectivity is illustrated.

With reference to Fig. 3, in Transcript A ("T#1"), the intron is not spliced out, and a translation stop codon results in a truncated protein designated sstr2a. In Transcript B ("T#2") the intron is spliced out, and translation proceeds into the second exon and an alternative translation stop coding resulting in the sstr2b protein which differs from sstr2a only in the short C-terminal tail. The positions of the PCR primers used to unambiguously identify sstr2 transcripts are also shown as (a) for an sstr2 primer set and (b) for an sstr2A primer set. The sizes of the resulting amplicons from the two different transcripts are 398 bp (T#1, sstr2 primer set), 87 bp (T#1, sstr2A primer set), 57 bp (T#2, sstr2 primer set), and no band (T#2 sstr2A primer set).

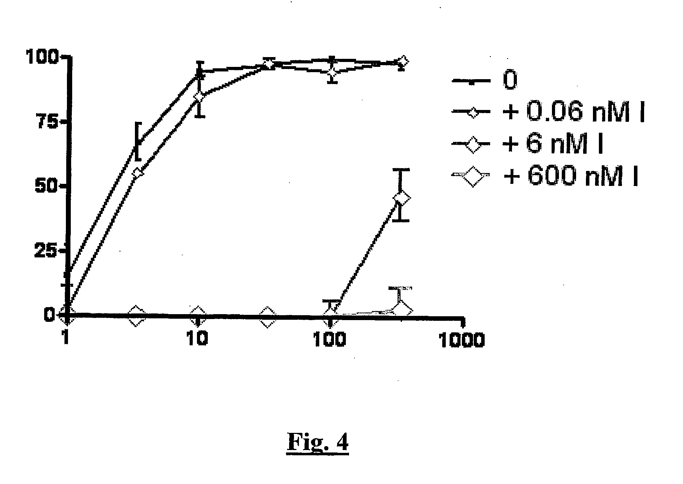

In Fig. 4, it can be seen that the absence of the antagonist (black line, small squares) BN83253 inhibits MCP-1 induced cell migration in a dose-dependent manner. As the concentration of the antagonist is increased (lighter grey, larger symbols) the BSCI activity of BN832653 is progressively lost.

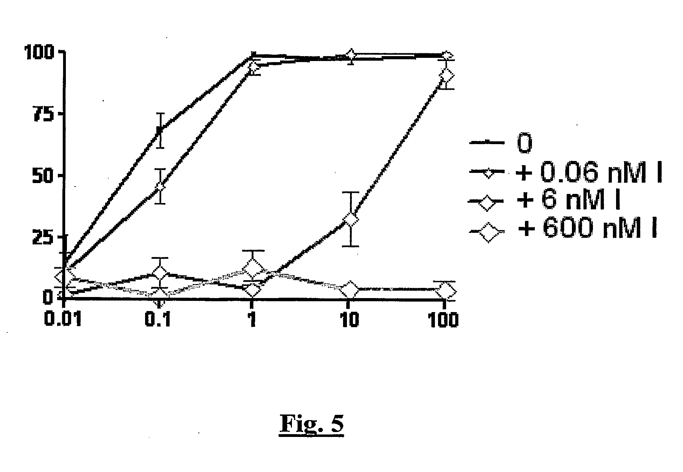

In Fig. 5, in the absence of the antagonist (black line, small squares) the valerolactam inhibits MCP-1 induced cell migration in a dose-dependent manner. As the concentration of the antagonist is increased (lighter grey, larger symbols) the BSCI activity of BN832653 is progressively lost. Note that this valerolactam BSCI is 20-50 fold more potent than BIM 83253 and as a result, higher concentrations of the competitive antagonist (BIM 23627) are required to abolish its BSCI activity than from NIM83253 (compare this graph with Fig. 4).

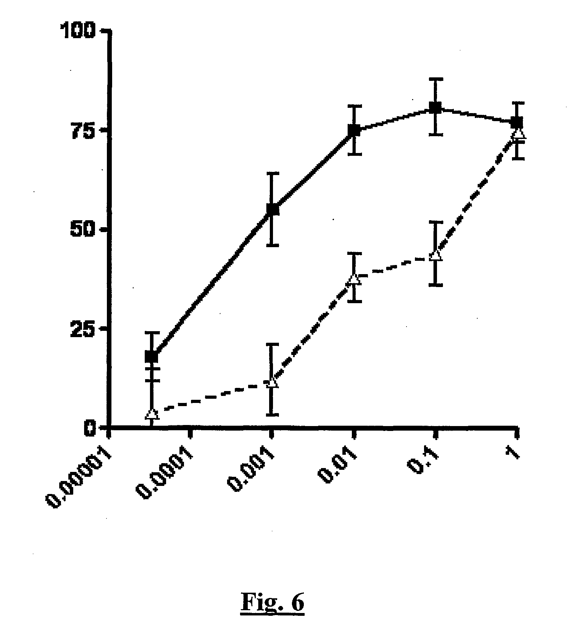

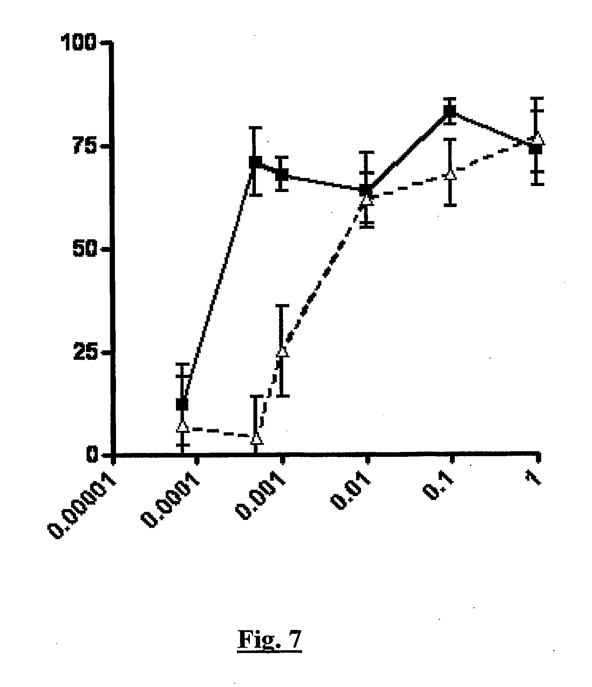

In both Figs 6 and 7, the BSCI inhibited TNF-alpha production in response to endotoxin in a dose-dependent manner (solid line, square symbols). The apparent potency of the BSCI was reduced by co-administration of BIM 23627 (dashed line, triangle symbols).

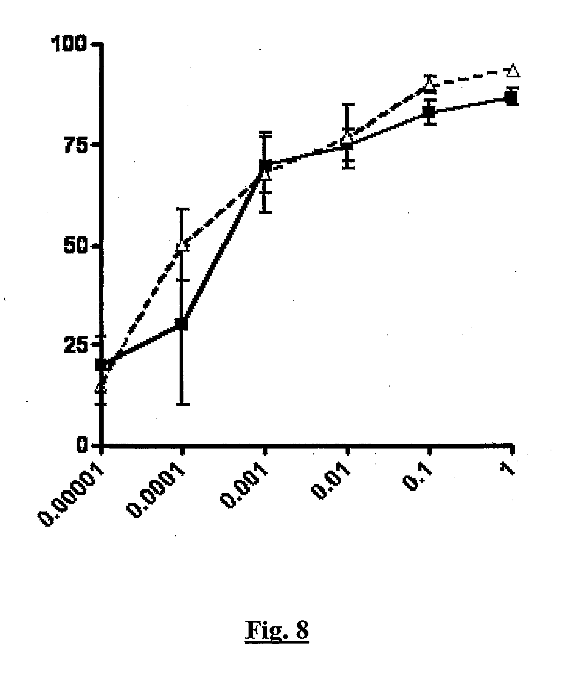

In Fig. 8, dexamethasone inhibited TNF-alpha production in response to endotoxin in a dose-dependent manner (solid line, square symbols). Co-administration of BIM 23627 (dashed line, triangle symbols) had no effect on the apparent potency of dexamethasone;

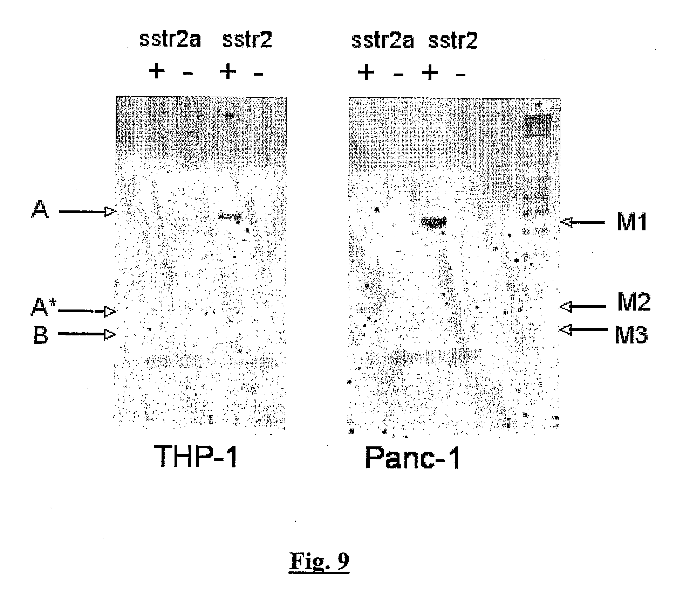

In Fig. 9, for both cell types two different primer sets were used (see Fig. 3), one which produces an amplicon ("A") only from the sstr2a transcript, the other which generates different sized amplicons ("A*" and "B") from sstr2a and sstr2b (designated "sstr2" here): In Fig. 9, M1 is 398 bp, M2 is 87 bp and M3 is 57 bp. The data confirm the expression of sstr2a in THP-1 cells, and provide no evidence for the presence of sstr2b.

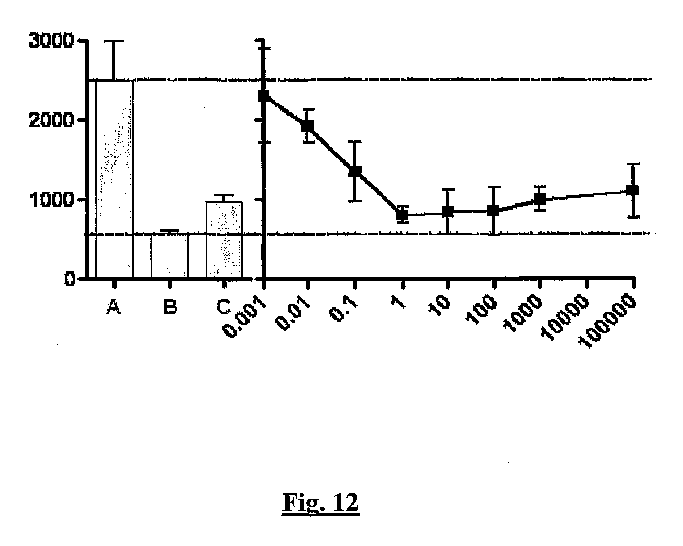

In Fig. 12, values are triplicate determinations (±SD) at each drug concentration. Competition with an excess of unlabelled BN83250 ("A", 10nM) 3H-BN83250) is shown as a control for non-specific binding, and determines the upper dashed line indicating specific binding. A negative control ("B", excess cold) is provided and determines the lower dashed line indicating non-specific binding, while the effect of the sstr2 antagonist BIM 23627 ("C"; 100µM) on BN83250 binding is shown for comparison.

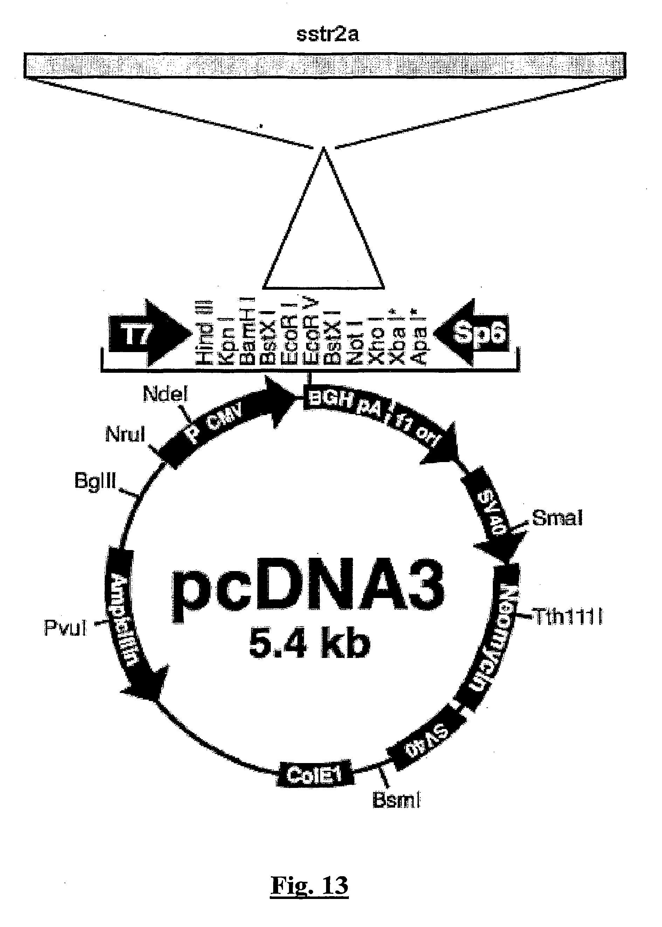

In Fig. 13, "*" in the polylinker denotes that there is an ATG upstream of the XbaI site. Note the "Ampicillin" and "Neomycin" resistance-encoding reading frames, used to select for the presence of the plasmid in bacteria and Jurkat cells respectively. The cDNA insert is expressed under the CMV promoter ("P CMV") in the eukaryotic system, and carries a canonical polyadenylation sequence ("BGH pA").

With reference to Fig. 14, (A) shows that BN83250 inhibits SDF-1α induced migration of Jurkat 3DB (albeit with a lower potency than is observed with THP-1 cells), but has little or no effect on the SDF-1α induced migration of the parental Jurkat T cell line. For each cell line, the level of cell surface staining for sstr2A, determined by flow cytometry is shown in frequency histograms (B) to (D).

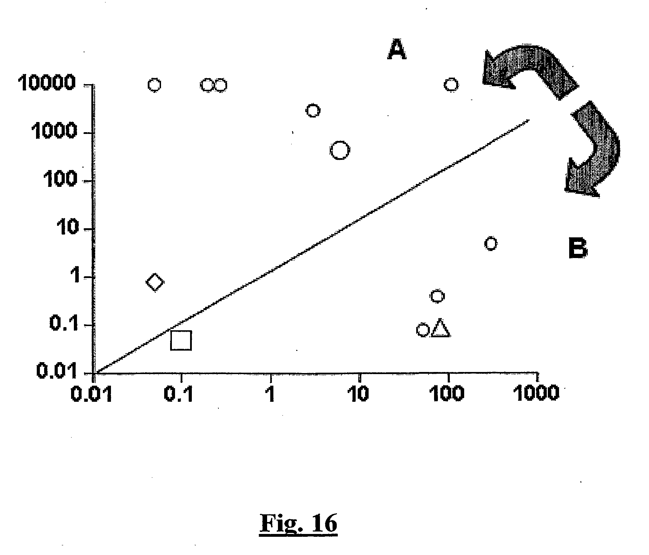

In Fig. 16, compounds with no effect on migration are plotted with a potency of 10µM so that they appear on the graph. BN83250 (open triangle), (S)-3-(2'2'-dimethylpropionoylamino)-tetrahydropyridin-2-one (open square), somatostatin (open diamond) and L-779,976 (open circle) can be identified from the graph. A line of identity where ED50 and Ka are equal is shown to emphasise that all known "BSCI-type" sstr2 agonists lie in the lower right, while all known "classical" sstr2 agonists lie in the upper left.

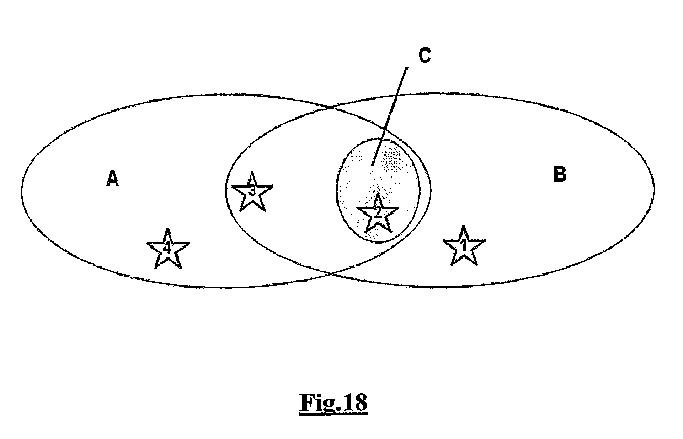

With reference to Fig. 18, we have demonstrated for the first time in the present disclosure that some BSCIs shown in oval B (including Peptide 3 derivatives and the aminocaprolactams and aminovalerolactams) are type 2 somatostatin receptor (sstr2) agonists (oval A). However, not all.sstr2 agonists have BSCI activity, and even among those which do the optimal subset are those which have higher potency as inhibitors of leukocyte migration than they do on "classical" targets of somatostatin, such as GH or glucose handling. Compounds in this "sweet spot" (grey oval labelled "C"), which we have dubbed "somatotaxins" have particularly promising properties as anti-inflammatory agents (since they will likely lack side-effects caused by the presence of "classical" sstr2 agonist activity). The method of the present invention is directed to identifying entirely novel members of this newly defined class. Examples of compounds which would fall in the various sections of this Venn Diagram, designated by stars, are as follows: Region 1: yohimban-16-amide or M3 chemokine binding protein; Region 2: Peptide 3, NR58-3.14.3, BN83253, BN83250 or (S)-3-(2'2'dimethylpropionylamino)-tetrahydropyridin-2-one;.Region 3: somatostatin or L779,976; Region 4: BIM23A1089 or BIM2-3120.

### Example 1 : Sstr2 antagonists block BSCI activity in vitro

In order to demonstrate that known peptide and aminolactam BSCI compounds act through the sstr2 receptor, we have employed a series of specific sstr2 antagonists. If the BSCI effects of various structurally distinct compounds are blocked by a series of antagonists against a particular receptor, then we conclude that BSCI activity depends upon binding to a receptor blocked by the antagonists used.

Here, we show that the ability of various BSCIs to block migration in response to chemokines (the canonical activity of BSCIs) is inhibited in vitro by several antagonists specific for the type 2 somatostatin receptor (sstr2).

*Methods:* The human myelomonocytic cell line THP-1 was used in these experiments, although similar results have also been obtained with human peripheral blood monocytes. Leukocyte migration was measured using the 96-well format micro Transwell^{RTM} assay system from Neuroprobe (Gaithersburg, MD, USA). In principle, this assay consists of two chambers separated by a porous membrane. The chemoattractant is placed in the lower compartment and the cells are placed in the upper compartment. After incubation for a period at 37°C the cells move towards the chemoattractant, and the number of cells in the lower compartment is proportional to the chemoattractant activity (relative to a series of controls).

Although a range of conditions for the assay are acceptable to demonstrate the chemokine-induced leukocyte migration (as well as the impact of BSCIs or the reversal of BSCI activity by sstr2 antagonists), nevertheless a specific example is hereby provided. The method was performed essentially as previously described (Reckless & Grainger (1999) Biochem. J. 340:803-11).

### Materials

The Transwell^{RTM} migration systems were manufactured by Neuroprobe, Gaithersburg, MD, USA.

The plates used were ChemoTx™ plates (Neuroprobe 101-8) and 30 µl clear plates (Neuroprobe MP30).

Geys' Balanced Salt Solution was purchased from Sigma (Sigma G-9779).

Fatty acid-free BSA was purchased from Sigma (Sigma A-8806).

MTT, i.e. 3-(4,5-dimethylthiazol-2-yl)-2,5-diphenyltetrazolium bromide, was purchased from Sigma (Sigma M-5655).

RPMI-1640 without phenol red was purchased from Sigma (Sigma R-8755).

The THP-1 cell line (European Cell culture Collection) were used as the leukocyte cell population.

### Test protocol

First, the cell suspension to be placed in the upper compartment was prepared. The THP-1 cells were pelleted by centrifugation (770 x g; 4 mins) and washed with Geys Balanced Salt Solution with 1mg/ml BSA (GBSS + BSA). This wash was then repeated, and the cells re-pelleted before being resuspended in a small volume of GBSS + BSA for counting, for example using a standard haemocytometer.

The volume of GBSS + BSA was then adjusted depending on the number of cells present so that the cells were at final density of 4.45 x 10⁶ cells per ml of GBSS + BSA. This ensured that there were 100,000 THP-1 cells in each 25µl of the solution to be placed in the upper chamber of the plate.

To demonstrate the reversal of BSCI activity against MCP-1 induced migration, it was necessary to prepare three lots of cells. The suspension of THP-1 cells at 4.45 x 10⁶ cells/ml was divided into three pots. To one pot the BSCI was added at an appropriate final concentration, in an appropriate vehicle (for example (S)- 3-(2',2'-dimethylpropanoylamino)-tetrahydropyridin-2-one was added at 1nM from a stock solution in GBSS). To the second pot, the BSCI was added at the same concentration as in the first pot, but additionally the sstr2 antagonist was also added (for example BIM 23454 [Tulipano et al. (2002) 143:1218-24] or BIM 23627 were added at 66nM final concentration from a stock solution in GBSS). To the third pot, GBSS + BSA plus vehicle as appropriate was added so that the volumes in three pots are equal, acting as a control.

Next, the chemoattractant solution to be placed in the lower compartment was prepared. MCP-1 was diluted in GBSS + BSA to give a final concentration of 25 ng/ml (after all additions). Note that, as recommended by Frow et al (Med. Res. Rev. 24:276-98) this is the concentration of MCP-1 which induces maximal migration in this system. This solution was divided into three pots, as for the cell suspension. To one pot, the BSCI was added to the same final concentration as was added to the cell suspension, in the second pot the BSCI and the sstr2 antagonist were both added at the same concentration as previously added to the cell suspension, while to the remaining pot GBSS + BSA plus vehicle as appropriate was added so that the volumes in the three pots were equal. Note that the volume of liquid that needs to be added to make the addition of the BSCI and the sstr2 antagonist compounds needs to be taken into account, when establishing the final concentration of MCP-1 in the solution for the lower compartment and the final concentration of cells in the upper compartment.

Once the chemoattractant solutions for the lower wells and cell solutions for the upper chambers were prepared, the migration chamber was assembled. 29 µl of the appropriate chemoattractant solution was placed into the lower well of the chamber. Assays were performed with at least triplicate determinations of each condition. Once all the lower chambers were filled, the porous membrane was applied to the chamber in accordance with the manufacturer's instructions. Finally, 25 µl of the appropriate cell solution was applied to each upper chamber. A plastic lid was placed over the entire apparatus to prevent evaporation.

The assembled chamber was incubated at 37 °C, 5% CO₂, for 2 hours. A suspension of cells in GBSS + BSA was also incubated under identical conditions in a tube: these cells were used to construct a standard curve for determining the number of cells that migrated to the lower chamber under each condition.

At the end of the incubation, the liquid cell suspension was gently removed from the upper chamber, 20 µl of ice-cold 20mM EDTA in PBS was added to the upper chamber, and the apparatus was incubated at 4°C for 15 mins. This procedure caused any cells adhering to the underside of the membrane to fall into the lower chamber.

After this incubation the filter was carefully flushed with GBSS + BSA to wash off the EDTA, and then the filter was removed.

The number of cells migrated into the lower chamber under each condition can then be determined by a number of methods, including direct counting, labelling with fluorescent or radioactive markers or through the use of a vital dye. Typically, we utilised the vital dye MTT. 3 µl of stock MTT solution were added to each well, and then the plate was incubated at 37 °C for 1-2 hours during which time dehydrogenase enzymes within the cells converted the soluble MTT to an insoluble blue formazan product that could be quantified spectrophotometrically.

In parallel, an 8-point standard curve was set up. Starting with the number of cells added to each upper chamber (100,000) and going down in 2-fold serial dilutions in GBSS + BSA, the cells were added to a plate in 25 µl, with 3 µl of MTT stock solution added. The standard curve plate was incubated along side the migration plate.
At the end of this incubation, the liquid was carefully removed from the lower chambers, taking care not to disturb the precipitated formazan product. After allowing to air dry briefly, 20µl of DMSO was added to each lower chamber to solubilise the blue dye, and absorbance at 595nm was determined using a 96-well plate reader. The absorbance of each well was then interpolated to the standard curve to estimate the number of cells in each lower chamber.

The MCP-1 stimulated migration was determined by subtracting the average number of cells that reached the lower compartment in wells where no MCP-1 was added from the average number of cells that reached the lower compartment where MCP-1 was present at 25ng/ml.

The impact of the BSCI was calculated by comparing the MCP-1-induced migration that occurred in the presence or absence of various concentrations of the BSCI.
Typically, the inhibition of migration was expressed as a percentage of the total MCP-1 induced migration that was blocked by the presence of the BSCI. For most compounds, a dose-response graph was constructed by determining the inhibition of MCP-1 induced migration that occurs at a range of different compound concentrations (typically ranging from 1nM to 1 µM or higher in the case of poorly active compounds). The inhibitory activity of each compound was then expressed as the concentration of compound required to reduce the MCP-1-induced migration by 50% (the ED₅₀ concentration). The impact of the sstr2 antagonist was calculated by comparing the MCP-1-induced migration occurring in the presence of the sstr2 antagonist and the BSCI with the MCP-1-induced migration occurring in the presence of the BSCI alone, at the same concentration of BSGI compound. If the sstr2 antagonist inhibited the BSCI activity of the BSCI compound then more migration was expected to have occurred in the wells treated with both sstr2 antagonist and BSCI than in the wells receiving BSCI alone. *Results:* MCP-1 (25ng/ml) induced substantial migration of the THP-1 cells (25,000 to 40,000 more cells migrate into the lower chamber in the presence of MCP-1 than in the control wells with no MCP-1). This migration was inhibited in a dose-dependent fashion by a wide range of BSCIs. In Fig. 4, the inhibition of MCP-1 induced THP-1 cell migration in response to increasing concentrations of the BSCI BN83253 (N-oleoyl-3-aminocaprolactam; Fox et al. (2005) J. Med. Chem. 48:867-74) is shown (small squares). In Fig. 5, the inhibition of MCP-1 induced THP-1 cell migration in response to increasing concentrations of the valerolactam BSCI (S)-3-(2'2'-dimethylpropionylamino)-tetrahydropyridin-2-one is shown (small squares).
In both cases, the presence of the sstr2 antagonist BIM23627 at 600nM (approximately 100-fold above the Ki for this compound at sstr2; Tulipano et al. (2002) 143:1218-24) completely prevented any BSCI-mediated inhibition (in other words, MCP-1 induced migration is restored to control levels by the presence of high dose BIM23627, irrespective of the concentration of BSCI which was added; triangles in Fig. 4 and Fig. 5).

At lower doses of BIM23627 (for example, at 6nM, which is close to the Ki for this compound acting at this receptor; mid-size diamond symbols in Fig. 4 and Fig. 5), the BSCI effect was inhibited at lower doses of BSCI, but at higher doses THP-1 induced cell migration was still inhibited. This is consistent with a competition between the BSCI and the sstr2 antagonist for binding to the same site. At low BSCI concentrations, the sstr2 antagonist binds, and no inhibition of migration is seen. As the BSCI concentration rises, increasingly sstr2 is occupied by BSCI and not sstr2 antagonist and the BSCI activity manifests itself.

At very low doses of BIM23627 (for example, at 0.06nM, which is approximately 100-fold lower than the Ki for this compound acting at sstr2; smaller diamonds in Fig. 4 and Fig. 5), the BSCI effect is seen at all concentrations of BSCI compound, although a small degree of reversal is seen at the lowest BSCI concentrations, again consistent with the competition between these agents for binding to the same site.

These results have been replicated a number of times using different BSCIs, including (S)-3-(adamantane-1-carbonylamino)-caprolactam (see W02006/016152) and Peptide 3 (Reckless & Grainger (1999) Biochem. J. 340:803-11). Furthermore, the same results have also been obtained using a different sstr2 antagonist (BIM 23454; Tulipano et al. (2002) 143:1218-24). As a result, we conclude that BSCI activity is mediated through sstr2 (or a closely related receptor which is inhibited by both of the antagonists used) for a wide range of BSCI compounds of very different structural classes, ranging from peptides to aminocaprolactams and third generation aminovalerolactams.

Similar experiments have also been performed using antagonists selective for other somatostatin receptors. For example, BIM23787 (a gift from Ipsen, Paris, France) which is an antagonist selective for sstr3 and sstr5, causes little (<10%) reversal of BSCI activity even at concentrations of antagonist >10-fold above the Ki for the target receptor.

### Example 2 : Sstr2 antagonists block BSCI activity in vivo

BSCI activity in vitro is mediated through sstr2 (or a closely related receptor which is inhibited by both BIM 23627 and BIM 23454). Next we aimed to demonstrate that the anti-inflammatory activity of BSCIs in vivo is also sstr2 dependent. Fortunately, BIM 23627 has been shown to be active in vivo, and is able to block classical sstr2 mediated signals, at least for a short time following acute high dose administration (Tulipano et al. (2002) **143:**1218-24). Consequently, provided that we adopted a model of acute inflammation, it would be possible to determine if the anti-inflammatory effects of BSCIs were inhibited by concomitant sstr2 blockade.

We selected the sub-lethal endotoxemia model in mouse, because this has been well characterised in our laboratory (see for example Fox et al. (2002) J. Med. Chem. 45:360-70; Fox et al. (2005) J. Med. Chem. 48:867-74). The extent of systemic inflammation in response to a single intraperitoneal injection of bacterial lipopolysaccharide (LPS) can be estimated by measuring serum TNF-alpha 2 hours post challenge. Furthermore, it is well known that BSCIs powerfully inhibit inflammation in this model (Fox et al. (2005) J. Med. Chem. 48:867-74).

*Methods:* Mice (CD1 strain; adult males; 6 per group) were treated with BSCI (in an appropriate vehicle) or with vehicle alone by an appropriate route. For example, BN83250 (see Fig. 1) was dosed by subcutaneous injection (since it is only poorly orally bioavailable), whereas (S)-3-(2'2'-dimethylpropionylamino)-tetrahydropyridin-2-one was dosed by oral gavage. Simultaneously, one set of mice at each BSCI concentration received BIM 23627 at 4mg/animal (120mg/kg) by sub-cutaneous injection (a treatment condition known to suppress classical sstr2-mediated signalling, such as GH release, in rats; Tulipano et al. (2002) **143:**1218-24). Between 30mins and 1hour after dosing (a period when a maximal plasma concentration of BSCI has been achieved), the inflammatory challenge was elicited by intraperitoneal injection of 750µg of LPS (Sigma; L4130 lot#024K4077; 900,000 EU/mg).

After a further 2 hours, the animals were sacrificed by CO₂ asphyxiation, and blood was drawn by cardiac puncture. Serum was prepared by conventional methods, and the level of TNF-alpha in serum was measured using an ELISA (R&D Systems; Murine Quantikine code# MTA00) performed in accordance with the manufacturer's instructions.

*Results:* Following analysis, the mean in each group of animals was reported (excluding any datapoint more than 3 standard deviations above or below the remaining points). As expected, LPS challenge induced a massive systemic inflammation with levels of TNF-alpha reaching an average of more than 3,000 ng/ml (compared with <10ng/ml in the serum from control animals which received no LPS challenge). This inflammation was dramatically inhibited by treatment with BSCI in a dose-dependent fashion, irrespective of the BSCI employed (Fig. 6 shows BN82350 [see Fig. 1]; Fig. 7 shows (S)-3-(2'2'-dimethylpropionylamino)-tetrahydropyridin-2-one [valerolactam]).

Co-administration of BIM 23627, the sstr2 antagonist that is active in vivo, markedly reduced the anti-inflammatory potential of the BSCI at all doses tested (dashed line, triangle symbols in Fig. 6 and 7). This observation was repeated twice for each BSCI in separate experiments. We estimate that the anti-inflammatory potency of BSCIs (irrespective of the BSCI which is adopted) is reduced by 10- to 100-fold by co-administration of BIM 23627. These results unequivocally demonstrate that BSCI activity in vivo is dependent on sstr2 (or a closely related receptor also inhibited by BIM 23627), exactly as we had previously observed in vitro (see Example 1).

In a separate series of control experiments, the effect of BIM 23627 on the anti-inflammatory activity of dexamethasone, a synthetic corticosteroid, which exerts its anti-inflammatory activity through a mechanism entirely unrelated to BSCIs, was determined. Dexamethasone reduced serum TNF-alpha levels (as a marker of systemic inflammation) to a similar degree to the BSCIs (compare Fig. 8 with Figs 6 and 7), although dexamethasone was slightly more powerful at the very high doses (inhibiting TNF-alpha by more than 90% compared to ∼80% inhibition at the highest doses of BSCIs) while BSCIs were slightly more potent (with statistically significant reductions in TNF-alpha at doses 3-10 fold lower than for dexamethasone). Nevertheless, despite their similar activities in this model, co-administration of BIM 23627 did not affect the anti-inflammatory potential of dexamethasone at all (dashed line and triangle symbols in Fig. 8). We conclude, therefore, that BIM 23627 does not have any non-specific effects on inflammation pathways and that BSCIs (but not other anti-inflammatory medicaments which work by different mechanisms) are highly dependent on sstr2 in vivo.

### Example 3 : Sstr2 expression is correlated with BSCI sensitivity

The use of multiple sstr2-specific antagonists demonstrates that BSCI activity depends on interaction with sstr2, or else to a different receptor (likely closely related to sstr2) that is also inhibited by the same antagonists. To further implicate sstr2 as the molecular target for BSCIs, we have measured the levels of sstr2 by flow cytometry and by qualitative PCR in a range of different leukocyte types with differing sensitivity to BSCIs. A correlation between the presence of sstr2 and sensitivity to BSCIs further strengthens the conclusion that BSCI activity requires binding to, and activation of, sstr2.

*Methods:* Flow cytometry, using an antibody specific for sstr2, was used to estimate relative levels of sstr2 expression on human peripheral blood leukocytes. Whole blood was treated with Erythrolyse (Serotec cat number: BUF04B) in accordance with the manufacturer's instructions. The resulting total leukocyte population was stained with a rabbit polyclonal anti human-sstr2 antibody (Sigma, Cat code S0694; 1µg/ml) followed by a anti-rabbit secondary antibody labeled with FITC (Sigma F9887; 50µg/ml) in parallel with a bank of monoclonal antibodies labeled with rPE against various leukocyte CD markers which could be used to unambiguously identify particular leukocyte subsets. After gating cell populations on the basis of their CD markers, the average level of sstr2 staining (with isotype control subtracted) was reported for triplicate determinations as mean ± SEM. THP-1 and Jurkat cell lines in culture were also analysed using the same protocol, except that the cells were not treated with Erythrolyse.

PCR was performed using the primer sets illustrated in Fig. 3 to distinguish the sstr2a and 2b transcripts. mRNA was prepared from THP-1 and Jurkat cells using the RNAqueous 4PCR kit (Ambion, Cat code #1914) in accordance with the manufacturer's instructions. The resulting mRNA preparations were then converted to cDNA using reverse transcriptase (RT), and the cDNA was then used to set up the following PCR reaction with sstr2 primers, dNTPs (Ambion), PCR buffer, and Taq polymerase in a total volume of 45 µl. The primer sequences were as follows: sstr2 forward 5' GCCAAGATGAAGACCATCAC 3' (SEQ ID NO: 3) and reverse 5' GATGAACCCTGTGTACCAAGC 3' (SEQ ID NO: 4). The PCR was performed using an Peltier PTC-200 thermal cycler in accordance with the manufacturer's instructions, using the following cycle times and conditions: 94°C for 2 min, followed by 30 cycles of 94°C, for 30 sec, 55°C for 30sec, 72°C for 1 min and finally 72°C for 10 min. At the end of the amplification, the products were separated on a 2% agarose gel in the presence of ethidium bromide and visualised on a UV transilluminator (Stratagene Eagle Eye II cabinet). Panc-1 cells, which are known to express sstr2 at high levels (Li et al. (2004) J. Surg Res. 119:130-137), were used as the positive control. Reactions without reverse transcriptase were used as the negative control (ensuring false positive signals were not obtained as a result of genomic DNA contaminating the mRNA preparation). Additionally, the primers were designed to span exon boundaries so that a genomic amplicon would be readily distinguished by size.

*Results:* Sstr2 was detected on several leukocyte populations, consistent with previous reports (Hiruma et al. (1990) Immunology 71:480-5; Tsutsumi et al. (1997) Cell. Immunol. 181:41-9; ten Bokum et al. (1999) J. Endocrinol. 161:167-75 and the references therein; Elliot et al. (1999) Eur. J. Immunol. 29:2454-63; Talme et al. (2001) Clin. Exp. Immunol. 125:71-9; Dalm et al. (2003) Am. J. Physiol. Endocrinol. Metab. 285:E344-53; Lichtenauer-Kaligis et al. (2004) Eur. J. Endocrinol. 150:565-77), and the relative levels, in arbitrary fluorescent units, are shown in Table 1 below. Interestingly, THP-1 cells (which are exquisitely sensitive to BSCIs) exhibit high levels of surface staining for sstr2 while Jurkat T cells (which are almost completely insensitive to BSCIs) show virtually undetectable levels of staining.

Human peripheral blood leukocytes exhibit staining for sstr2 that is generally intermediate between the two cell lines. Granulocytes (predominantly neutrophils) and monocytes express significant levels of sstr2, and both human peripheral blood leukocyte types are known to be sensitive to BSCIs. T lymphocytes (CD3+) express lower, but still detectable levels, which are likely similar on both CD4+ (helper) and CD8+ (killer) T cell populations, since the entire CD3+ population was homogeneously stained. In contrast, sstr2 was not detected on the B lymphocyte population, a cell type that is insensitive to BSCIs (consistent with the lack of effect of BSCIs on antibody generation in response to a neoantigen in vivo).

**Table 1. Relative Levels of sstr2 Cell Surface Staining, Estimated by Flow Cytometry, in Various Leukocyte Populations.**

| **Cell type** | **Markers** | **Person A** | **Person B** |
|---|---|---|---|
| Granulocytes | CD15, SSC^{a} | 46 ± 3 | 28 ± 5 |
| Eosinophils | CCR3 | 42 ± 7 | 25 ± 9 |
| T lymphocytes | CD3 | 18 ± 2 | 13 ± 4 |
| B lymphocytes | B220 | 0 ± 3 | 0 ± 2 |
| Monocytes | CD14 | 39 ± 6 | 24 ± 3 |
| THP-1 cell line | *Not applicable* | 358 ± 36 | |
| Jurkat T cell line | *Not applicable* | 1 ± 1 | |

| | | | |
|---|---|---|---|
| ^{a}SSC indicates gating on the basis of side scatter, since granulocytes have characteristically high optical dispersion properties as a result of their cytoplasmic granules. | | | |

The presence of mRNA for sstr2 was confirmed in THP-1 cells (Fig. 9) and shown to be absent in Jurkat T cells by qualitative PCR. This confirms the expected specificity of the antibody reagent used in these studies. Furthermore, we have demonstrated that the majority of the sstr2 present in THP-1 cells is of the sstr2a isoform (based on the splicing pattern of the mRNA in these cells; Fig. 3 & 9). It is not known whether the antibody used for the flow cytometry experiments distinguishes between the sstr2a and sstr2b isoforms, but the results presented strongly suggest the antibody can detect the sstr2a isoform.

We conclude that the presence of sstr2 on the leukocyte plasma membrane correlates with sensitivity to BSCIs, and note in particular that cells without detectable sstr2 expression are insensitive to BSCI-mediated inhibition of chemokine-induced cell migration.

### Example 4 : BSCIs bind to sstr2

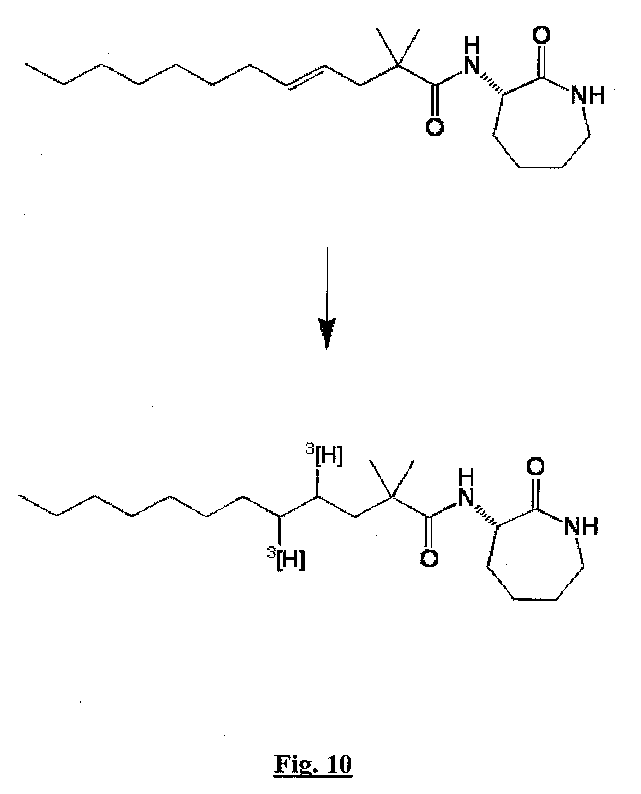

We have investigated the molecular interaction between BSCIs and the sstr2 receptor using conventional equilibrium binding studies employing labelled BSCI ligands. For these studies we have employed the aminolactam BSCI BN83250, which can readily by synthesized in a form labelled with tritium (that is [³H]) by catalytic hydrogenation of an unsaturated precursor (Fig. 10). The unsaturated precursor, (S,E)-3-(2',2'-dimethyldodec-4'-enoylamino)-caprolactam was synthesised as previously described (W02005/053702).

*Methods:* [³H]-BN83250 was synthesesized from (S,E)-3-(2',2'-dimethyldodec-4'-enoylamino)-caprolactam by catalytic hydrogenation using tritium gas over a palladium catalyst (CRB Ltd, Cleveland, UK), yielding the tritiated product at a concentration of 270µM in ethanol with a specific activity of 220Ci/mmol. The (S,E)-3-(2',2'-dimethyldodec-4'-enoylamino)-caprolactam was prepared as follows: (*S,S*)-3-amino-caprolactam hydro-pyrrolidine-5-carboxylate (10 mmol) and Na₂CO₃ (30 mmol) in water (30 ml) were added to a solution of 2,2-dimethyl-dodec-4-enoyl chloride (10 mmol) in dichloromethane (30 ml) at ambient temperature and the reaction was stirred for 12 hours. The organic layer was then separated and the aqueous phase was extracted with additional dichloromethane (2 x 25 ml). The combined organic layers were dried over Na₂CO₃ and reduced *in vacuo.* The residue was purified by silica column chromatography (1:1 EtOAc: hexanes to EtOAc) to give (*S,E*)-3-(2',2'-dimethyl-dodec-4'-enoyl)amino-caprolactam as a colourless oil (2.12 g, 63%). The radiolabelled compound was stored in the dark at 4°C until required. Prior to use the specific activity was adjusted by addition of unlabelled BN83250.

For the analysis of binding, individual reactions were set up (in triplicate for each condition), with 1x10⁶ THP-1 cells in Gey's Balanced Salt Solution, 10nM [³H]-BN83250 (at 50 Ci/mmol) and various concentrations of (S)-3(2'2'-dimethylpropionoylamino)-tetrahydropyridin-2-one (10pM to 10µM). Excess (100µM) unlabelled BN83250 was used to determine the level of non-specific binding. After 2 hours at 4°C (conditions previously shown to achieve equilibrium binding, and which prevent uptake of the radioligand that would result in an over-estimate of binding) excess label was washed away by centrifugation (3 washes with 5ml of Gey's Balanced Salt Solution) and the bound radioactivity was determined by scintillation counting of the solubilised cell pellet.

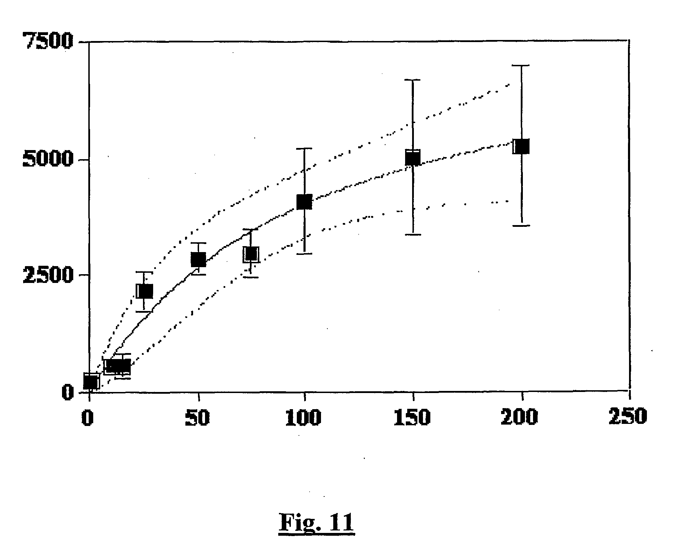

*Results:* [³H]-BN83250 shows specific, competable binding to THP-1 cells under equilibrium conditions. Scatchard analysis of the binding is consistent with a single binding site present at 100,000 to 200,000 sites per cell, with an affinity of approximately 80nM (Fig. 11). The specific binding of BN83250 to THP-1 cells is inhibited by 60-80% (depending on the experiment) by an excess (100µM) of the sstr2 antagonist BN23627 (Fig. 12). Very little specific binding (<20,000 sites per cell) was detected on Jurkat T cells, which lack sstr2, but specific binding was restored following stable transfection with a vector expressing sstr2 (see Example 5 for the preparation of a stable Jurkat T cell line expressing sstr2). Taken together, these results demonstrate that this binding site is sstr2, and is the binding interaction responsible for the observed BSCI activity of BN83250.

The effect of (S)-3-(2'2'-dimethylpropionoylamino)-tetrahydropyridin-2-one, which we have previously described as one of the highest potency BSCIs (WO2009/016390), competing at this binding site is shown in Fig. 12. (S)-3-(2'2'-dimethylpropionoylamino)-tetrahydropyridin-2-one competes with [³H]-BN83250 for binding at sstr2 in a dose-dependent manner with an ED50 of approximately 50pM and a Hill Slope of approximately -1.0, suggesting a simple mode of competitive binding. High levels of (S)-3-(2'2'-dimethylpropionoylamino)-tetrahydropyridin-2-one (1nM and above) inhibit BN83250 binding by 80-90% suggesting that the two molecules share an essentially identical binding site. Other BSCI compounds tested, including Peptide 3 (Reckless & Grainger (1999) Biochem. J. 340:803-11), NR58-3.14.3 (Reckless et al. (2001) Immunology 103:244-54; WO99/12968), (S)-3-(adamantane-1'-carbonylamino)-caprolactam (W02006/016152) and (S)-3-(1'-methylcyclohexyl-1'-carbonylamino)-caprolactam (WO2006/134384) also compete with BN83250 binding to sstr2 to a similar extent.

The apparent affinity constant for (S)-3(2'2'-dimethylpropionoylamino)-tetrahydropyridin-2-one binding to THP-1 cells is consistent with the picomolar ED50 for BSCI activity in the in vitro migration assay (see Example 1) but is several orders of magnitude lower than the ED50 for classical sstr2 agonist activity (see Example 7). We conclude that all the families of BSCIs described to date bind to sstr2, and that this interaction is responsible for the BSCI activity of the compounds.

### Example 5 : Expression of sstr2 confers BSCI sensitivity on cell lines

Here, we demonstrate that (forced) expression of the sstr2 receptor is both necessary and sufficient to confer susceptibility to BSCIs. We have already shown that cell-surface expression of sstr2 determined by flow cytometry correlates with sensitivity to BSCIs, such that cells with little or no sstr2 are insensitive to BSCIs, while cells with significant levels of sstr2 are susceptible to BSCI-mediated inhibition of chemokine-induced migration. Higher levels of sstr2 are associated with increased potency of the effect of BSCIs. However, this correlation does not conclusively demonstrate that sstr2 is responsible for the BSCI activity.

Using recombinant DNA techniques, we have forced ectopic expression of sstr2 in a leukocyte cell line, the Jurkat T cell line, which does not normally express sstr2. The level of expression achieved was determined by flow cytometry. Sensitivity of the stably transfected cell line to BSCIs was then determined, and compared with the parental Jurkat T cell line.

*Methods:* Total mRNA was prepared from Panc-1 cells (a human pancreas cell line which is well known to express high levels of sstr2) using the RNAqueous 4PCR kit (Ambion, Cat code #1914) in accordance with the manufacturer's instructions. The mRNA was then reverse transcribed (using the RETROscript kit; Ambio) to yield a cDNA preparation. A full length cDNA of the sstr2 gene was then obtained by PCR (using a Peltier PTC-200 thermal cycler using the following cycle times and conditions: 94°C for 2 min, followed by 30 cycles of 94°C, for 30 sec, 55°C for 30sec, 72°C for 1 min and finally 72°C for 10 min), and isolated by preparative gel electrophoresis. The sequence of the sstr2 cDNA was confirmed by sequencing. The sstr2 cDNA was treated with EcoRI and Xhol to generate sticky ends, which allowed directional cloning into the polylinker of the plasmid pcDNA3.1+ (Invitrogen) followed by ligation (10µl ligation reactions were run with 400 T4 DNA ligase (New England Biolabs) and the maximal amounts of DNA with an insert:plasmid ratio of 3:1 in ligase buffer incubated overnight at 13°C). The resulting vector (see Fig. 13) was used to transform XL-10 Ultracompetent cells (Stratagene) using standard techniques. Plasmid DNA was prepared using a MiniPrep kit (Wizard Plus SV Minipreps DNA purification System; Promega) in accordance with the manufacturer's instructions for transfection into Jurkat T cells.

Jurkat T cells were grown in RPMI + 10% foetal calf serum (FCS), and subcultured by 1:3 dilution in fresh medium every 4 days. Jurkat T cells in logarithmic growth phase 48 hours after subculture were washed by centrifugation and resuspended in serum-free RPMI medium at 7.5 x 10⁵ cells per ml. A mixture of DNA and transfection reagent (2µg of DNA; 3µl Fugene 6 transfection reagent; Roche) was then added and the cells were incubated at 37°C for 48 hours. The cells were then washed three times by centrifugation in RPMI + 10% FCS, before being suspended in fresh medium at 1 x 10⁵ cells per ml. 68 ml of the resulting cell suspension was then plated out in a total of 226 wells in 96-well plates (Corning). After 48hrs, G418 (0.9mg/ml Active G418) was added to the medium to select for transfectants, and the cells were expanded by repeated subculture, keeping the populations from individual wells (which were considered clonal or oligoclonal expansions) separate. Once the cell populations had been expanded into 25cm² flask (approximately 5 x 10⁶ cell in total), a small sample was removed and tested for expression of sstr2 by flow cytometry.

A number of individual cell lines were obtained in this way, which expressed significant levels of sstr2 on the cell surface, detected by flow cytometry using the human-sstr2 antibody (Sigma, S0694; see Example 3 for details of the flow cytometry protocol used). Of these, one line (designated Jurkat-D3B) was found to express high levels of sstr2 comparable to peripheral blood monocytes (the highest expressing cell population in healthy human peripheral blood; see Example 3), although the levels of sstr2 in Jurkat-D3B were still significant less than in THP-1 cells (Fig. 14).

*Results:* The level of the SDF-1α receptor CXCR4 was similar on Jurkat-D3B and the parental Jurkat T cell line. Furthermore, the Jurkat-D3B cells responded to SDF-1α in a Transwell^{RTM} chemotaxis assay (see Example 1 for the methodology employed) over a similar concentration range to the parental cell line. We conclude that the Jurkat T cells and the Jurkat-D3B cells exhibit identical chemokine-induced migration under the conditions of this experiment, and consequently are suitable for comparison of their sensitivity to BSCIs.

Jurkat T cells and Jurkat-D3B cells were induced to migrate in response to 100ng/ml SDF-1α (R&D systems; the concentration of SDF-1α which induced maximal cell migration in both lines). The BSCI BN83250 was then added at various concentrations between 0nM and 1000nM (each in triplicate) to both the upper and lower compartments (at the same concentration in each compartment, so as not to create a gradient of BSCI concentration). DMSO (1% final concentration) was used as the vehicle, and was present in all wells at equal concentration.

Consistent with previous results (see Example 3), the parental Jurkat T cells, which express low or undetectable levels of sstr2 (Fig. 14), were virtually insensitive to BN83250 (the ED50 was considerably abovel µM, compared to an ED50 for the this BSCI of <1nM on sensitive cells, such as the THP-1 cell line). In contrast, Jurkat-D3B cells, which express moderate levels of sstr2 (Fig. 14), were substantially more sensitive to BN83250 (Fig. 14). The ED50 for BN83250 on Jurkat-D3B was approximately 10nM, representing more than 100-fold increase in BSCI sensitivity as a result of the ectopic expression of sstr2. Transfection and selection for pcDNA3.1 with no insert generated Jurkat T cell lines that were similarly insensitive to BN83250 as the parental Jurkat T cells.

Jurkat-D3B were also sensitive to inhibition with other, structurally distinct, BSCIs including the peptide NR58-3.14.3 and the valerolactam (S)-3-(2'2'-dimethylpriopionoylamino)-tetrahydropyridin-2-one, although in each case the potency was significantly lower than on THP-1 cells. This likely reflects the lower levels of sstr2 expression achieved in Jurkat-D3B compared with THP-1 cells (Fig. 14). It is well known that potency of response to pharmacologic agents can correlate with the level of receptor expression (as a result of so-called "spare receptors"). Our data are consistent with the proposal that sensitivity to BSCI depends on the level of cell surface sstr2 expression.

We conclude that sstr2 expression is both necessary and sufficient to confer sensitivity to BSCIs on leukocyte cells.

### Example 6 : Conventional sstr2 agonists do not possess BSCI activity in vitro

Various structurally distinct compounds with BSCI activity have been shown to act through sstr2 (see examples 1 through 5 which confirm sstr2 as the molecular target of action of these BSCIs). To determine whether all agonists at sstr2 have BSCI activity, or alternatively whether the compounds functionally identified as BSCIs are a special class of sstr2 agonists, we tested a range of classical somatostatin receptor agonists for BSCI activity in vitro.

*Methods:* Leukocyte migration assays were performed exactly as described in Example 1. Briefly, THP-1 cells were induced to migrate in response to 25ng/ml MCP-1 in the ChemoTx™ 96-well plate migration system (Neuroprobe Inc., Gaithersburg, MD). Various classical sstr2 agonists were added to different wells (in triplicate), at equal concentration in both the upper and lower compartments (exactly as in the experiments to demonstrate BSCI activity of acylaminolactams, described previously; Reckless & Grainger (1999) Biochem. J. 340:803-11; Fox et al. (2002) J. Med. Chem. 45:360-70; Fox et al. (2005) J. Med. Chem. 48:867-74). After 2 hours at 37°C, the number of cells migrated into the lower compartment is determined using the vital dye MTT and interpolation of a standard curve, all exactly as described in Example 1.

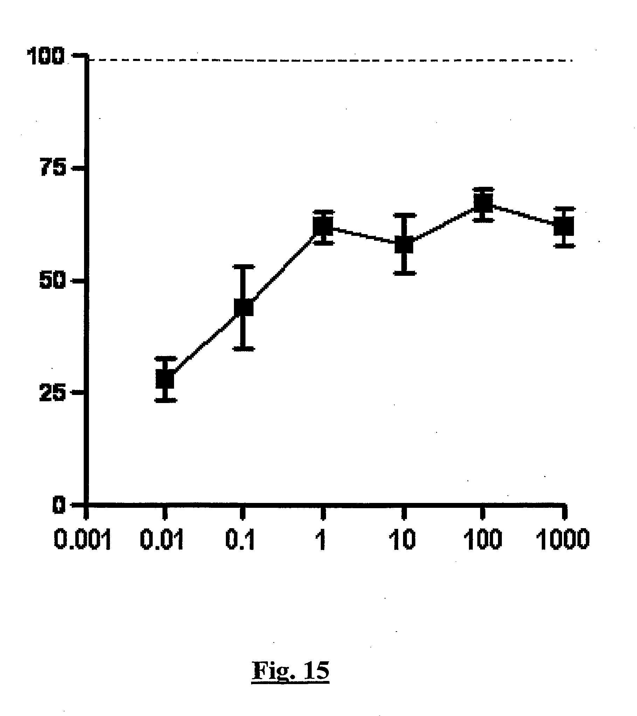

*Results:* Somatostatin (sst14), a natural peptide ligand at sstr2, was found to only partially replicate the BSCI effect (Fig. 15). Most notably, the maximal inhibition of leukocyte migration achieved even at maximal doses of sst14 (1 µM) was between 40 and 60% depending on the particular experiment. At concentrations of sst14 above 10nM, all the sstr2 receptors on the cells will be occupied, so this failure to completely block migration represents a qualitative difference in the way sstr2 signals in response to sst14 compared with the BSCIs (as illustrated diagrammatically in Fig. 2).

Furthermore, sst14 only inhibits leukocyte migration at concentrations above the binding constant for sstr2 binding (approximately 0.05nM), whereas all the BSCIs tested to date inhibit leukocyte migration at concentrations below their binding affinity. These observations suggest that BSCIs induce a more powerful signal (with respect to inhibiting leukocyte migration) than do classical sstr2 agonists, such as the natural ligand sst14. As a result, BSCIs need occupy only a tiny percentage of the cell surface receptors to maximally inhibit leukocyte migration, while conventional sstr2 agonists must occupy essentially all the receptors, and still do not achieve maximal inhibition.

Similar experiments were performed using the alternatively processed sst28 natural ligand, with similar results. Leukocyte migration was only inhibited at concentrations above the binding affinity at sstr2, and the maximal inhibition was only 40-60% even at very high doses. Again, similar results were obtained using the synthetic somatostatin analog lanreotide (Prevost et al. (1991) Endocrinology 129:323-9), which is used clinically to suppress growth hormone release in acromegaly.

We also tested cortistatin (de Lecea et al. (1996) Nature 381:242-5), a more recently described somatostatin analog whose receptor binding characteristics have not yet been fully determined. It is possible that cortistatin represents a different class of natural ligands at sstr2. However, we found that cortistatin was very similar in its effects on leukocyte migration (inhibiting only ∼50% of migration even when used at very high doses) to the somatostatins, but it was much less potent (ED50 = ∼ 75nM; Table 2). To date, therefore, no natural ligand at the sstr2 receptor can replicate the powerful BSCI activities of the acylaminolactams acting at the same receptor.

Finally, we tested an array of synthetic sstr2 selective and specific agonists provided to us by Ipsen (Paris, France) and referred to as "BIM" numbers in Tables 2 and 3 below. Somatostatin binds to all of the somatostatin receptors with broadly similar affinity, and it is possible that the weak BSCI-like activity was due to opposing signals generated through a different receptor. However, with the exception of BIM23065, which inhibited leukocyte migration at 10µM (but not at lower doses), none of these selective or specific conventional sstr2 agonists displayed any significant activity in the leukocyte migration inhibition assay (Table 2).

**Table 2. Binding constant ("Ka") at sstr2 for various known or suspected sstr2 agonist compounds, and ED50 for inhibition of MCP-1 induced THP-1 cell migration. The maximum inhibition ("Max effect") observed at the highest dose tested (1-10µM) is also shown. The affinity of cortistatin at sstr2 has not been unambiguously reported. BIM23065 achieved 85% inhibition of cell migration at the highest concentration available to be tested (10µM) so it is unclear whether this represents the maximum effect achievable. BIM23A387 is described in Pawlilcowsky et al. (2007) J. Physiol. Pharmacol. 58:179-88, and BIM23120 is described in Ferrante et al. (2006) Endocrine-Related Cancer 13:955-62.**

| **Agent** | **Ka (sstr2)** | **ED50** | **Max effect** |
|---|---|---|---|
| Sst-14 | 0.05nM | 0.8nM | 40-60% |
| Sst-28 | 0.1nM | 0.9nM | 40-60% |
| Cortistatin | ? | 75nM | 40-60% |
| BIM23065 | 3nM | 3000nM | 75%? |
| BIM23032 | 109nM | No effect | No effect |
| BIM23A108 | 0.05nM | No effect | No effect |
| BIM23A387 | 0.2nM | No effect | No effect |
| BIM23120 | 0.28nM | No effect | No effect |

Taken together, these results clearly demonstrate that BSCIs are a special class of sstr2 agonists, and that most compounds (including the natural ligands) are considerably less able to inhibit leukocyte migration (and hence exhibit anti-inflammatory activity in vivo) than those agents known to be BSCIs. The extent to which BSCIs differ from classical agonists is illustrated in Fig. 16, which plots affinity at sstr2 versus ED50 for inhibition of chemokine-induced leukocyte migration. The BSCIs cluster together, and are markedly separated from the conventional agonists. It is, therefore, clearly possible to identify unambiguously new, structurally distinct families of BSCIs (which have no structural resemblance to any known BSCI) using this combination of tests.

Furthermore, it is evident that the method demonstrated here differentiates BSCIs from the weak, and clinically less useful, anti-inflammatory activity which has already been proposed for some somatostatin receptor agonists, including the natural ligand sst14 and its clinical analog lanreotide (Paran & Paran (2003) Curr. Opin. Invetig. Drugs 4:578-82; Sener et al. (2005) Peptides 26:493-9; Gonzales-Rey et al. (2007) Ann. Rheum. Dis. 66:582-8 and the references therein; Armani et al. (2007) J. Leukoc. Biol. 81:845-55; Cury et al. (2008) Dig. Dis. Sci. 53:2516-20).

Existing methods for identifying BSCIs essentially depended on random screening using a functional assay to identify hits, together with conventional medicinal chemistry to obtain further leads around the original hit structures based on defining structure activity relationships. The method described here is considerably more useful because it allows the replacement of the resource-intensive low-to-medium throughput functional assay with a high throughput, low cost molecular screen. In this implementation of the method of the invention, compounds are first selected on the basis of their interaction with sstr2 and then the relatively small number of positive hits are further screened for leukocyte inhibition activity to identify new BSCIs. This application is further illustrated in Examples 8 and 9 below.

### Example 7 : BSCIs inhibit leukocyte migration more potently than other conventional sstr2 targets

BSCIs are agonists at the sstr2 receptor, but not all sstr2 receptor agonists demonstrate BSCI activity. Here we have investigated whether the opposite is also true: do BSCIs possess potent sstr2-agonist like activity assessed by other functional assays?

Two different assays for "classical" sstr2 functions were adopted: (1) cultured foetal rat pituitary cells secreted growth hormone which is inhibited by sstr2 agonists in a dose-dependent fashion and (2) selected glucose-sensitive sub-clones of the human Panc-1 cell line secrete insulin which is inhibited by sstr2 agonists in a dose-dependent fashion.

*Methods:* Cultures of fetal rat pituicytes were established exactly as previously described (Murray et al. (2004) J. Clin. Endocrinol. Metab. 89:3027-32) by enzymatic dispersion. After 48hrs in low glucose DMEM plus 10% FCS, cells in 48 well plates (5 x 10⁴ cells per well) were washed for 4hrs in serum-free DMEM and then incubated with test agents at various concentrations for a further 24hrs in serum-free DMEM. At the end of this period, the conditioned medium was harvested, any cell debris removed by centrifugation and assayed for GH using a commercially available ELISA (Linco; cat# EZRMGH-45K) in accordance with the manufacturer's instructions.

Glucose-sensitive sub-clones of the human Panc-1, a cell line derived from a pleiomorphic epithelioid carcinoma of the exocrine pancreas, were obtained essentially as described by Hamil et al. (2008) Biomed. Sci. Instrum. 44:441-6. Briefly, Panc-1 cells were subcultured in 24 well plates, and the medium was supplemented with an additional 5% glucose. After 72 hrs, wells which showed the most marked changes towards the glucose-sensitive round phenotype were selected and expanded in normal medium. The resulting sub-lines were then tested for glucose sensitivity by preparing wells of a 96-well plate (5 x 10⁴ cells per well) which were then exposed to cultured medium containing a range of glucose concentrations (low glucose DMEM, DMEM, DMEM + 1% glucose, DMEM ± 2.5% glucose and DMEM + 5% glucose). After 24hrs incubation, the conditioned medium was collected and assayed for insulin secretion by ELISA (Linco; cat# EZHI-14K) in accordance with the manufacturer's instructions. Using the clone (Panc-2H4) which had the highest glucose sensitivity (insulin secretion increased 3.4 fold in 5% glucose compared to low glucose DMEM) and which was shown to express sstr2 by flow cytometry using the methods described in Example 3, we tested the ability of various sstr2. agonists to modulate glucose-dependent insulin secretion. Further 96 well plates were prepared using Panc-2H4 (5 x 10⁴ cells per well) in DMEM. After 24hrs, the medium was switched to DMEM + 5% glucose containing various test agents at various concentrations (triplicate wells per condition). After a further 24hrs, the conditioned medium was harvested and the concentration of insulin was determined by ELISA as described above.

In both assays, the absence of an effect of the test compounds (at the highest concentration tested) on the ELISAs was demonstrated by including an additional control well in the ELISA exposed to the second-highest concentration standard in the presence of each test compound. Results were reported as the mean ± SD, expressed as the fold change from cells in the presence of vehicle only.

*Results:* As expected, classical sstr2 agonists including the natural ligand somatostatin-14 caused a dose-dependent decrease in GH secretion from rat fetal pituicytes, maximal at 10nM with a 64 ± 9% inhibition (Table 3). Classical agonists had a similar effect on insulin secretion by Panc-2H4 (Table 3), although the maximal effect was smaller (43 ± 7% inhibition). In both assays, the concentrations required to suppress GH or insulin secretion were consistent with the known affinities of the various agents for binding to sstr2.

In marked contrast, these classical sstr2 agonists did not inhibit the.migration of either THP-1 cells or rat peripheral blood leukocytes in response to chemokines, or else did so only at concentrations substantially higher than those required to inhibit GH and/or insulin secretion (Table 3).

A range of structurally distinct BSCIs, including the peptides Peptide 3 and NR58-3.14.3, as well as the aminocaprolactams BN83250 and BN83470 and the valerolactam (S)-3-(2'2'-dimethylpropionoylamino)-tetrahydropridin-2-one inhibited GH release only at much higher concentrations (Table 3), and the maximal effect was smaller. None of these agents inhibited insulin secretion by Panc-2H4 even at the highest concentrations tested. However, as expected these agents potently inhibited the migration of THP-1 cells and rat peripheral blood leukocytes in response to chemokines (Table 3).

Taken together the results in Table 3 demonstrate that compounds which bind to sstr2, and which act as agonists at that receptor, can be readily divided into two classes. One group of agents (designated for the purposes of this application as "classical" sstr2 agonists) inhibit GH secretion and insulin secretion more potently and powerfully than they inhibit chemokine-induced leukocyte migration. The second, novel, grouping of agents (designated for the purposes of this application as "BSCI-type" sstr2 agonists or somatotaxins) inhibit chemokine-induced leukocyte migration more potently and powerfully than they inhibit chemokine-induced leukocyte migration.

**Table 3. Comparison of the potency of various sstr2 agonists on "classical" versus "BSCI" targets. The potency (ED50 in nM) and power (maximum effect as a percentage suppression compared to vehicle) of the effect of 12 structurally distinct sstr2 agonists in four separate assays is shown. Two assays measure classical sstr2 dependent responses (GH suppression and insulin suppression) and two assays measure BSCI activity through sstr2 (inhibition of leukocyte migration in response to chemokines). The relative potency of these two categories of responses allows the sstr2 agonists to be functionally classified according to the method of the present invention: the first 7 agents are classified as "classical" sstr2 agonists and the last 5 agents are classified as "BSCI" sstr2 agonists. Abbreviations: SST = somatostatin; n.d. = not determined; "Valerolactam" = (3)-3-(2'2'-dimethylpropionoylamino)-tetrahydropyridin-2-one. BN83470 = (S)-3-(admantane-1'-carbonylamino)-caprolactam.**

| **Compound** | **Rat pituicyte GH suppression assay** | | **Human Panc-2H4 insulin suppression assay** | | **Rat chemokine-induced PBMC migration assay** | | **Human chemokine-induced THP-1 migration assay** | | **"Classical" : "BSCI" potency ratio** | |
|---|---|---|---|---|---|---|---|---|---|---|
| | **ED50 (nM)** | **Max effect (%)** | **ED50 (nM)** | **Max effect (%)** | **ED50 (nM)** | **Max effect (%)** | **ED50 (nM)** | **Max effect (%)** | **Rat** | **Human** |
| SST-14 | 0.09 | 64 | 0.16 | 43 | 1.4 | 41 | 0.8 | 52 | 15 | 5 |
| SST-28 | 0.17 | 73 | 0.14 | 51 | 1.2 | 44 | 0.9 | 38 | 7 | 6 |
| BIM23065 | 4.2 | 52 | 7.9 | 38 | 1,000 | 71 | 3,000 | 75 | 238 | 380 |
| BIM23A108 | 0.10 | 54 | 0.15 | 25 | >1,000 | 10 | >1,000 | <10 | >10,000 | >6,666 |
| BIM23A387 | n.d | n.d | 0.56 | 33 | n.d. | n.d. | >1,000 | <10 | n.d. | >1,786 |
| BIM23120 | 0.88 | 60 | 3.9 | 37 | n.d. | n.d | >1,000 | <10 | n.d. | >256 |
| L-779,976 | 2.9 | 55 | 17 | 40 | n.d. | n.d. | 450 | 65 | n.d. | 27 |
| NR58-3.14.3 | 420 | 30 | >1,00 0 | 10 | 7.3 | 100 | 3.0 | 100 | 0.017 | <0.003 |
| BIM58187 | 550 | 42 | >1,00 0 | <10 | 15 | 100 | 6.9 | 100 | 0.027 | <0.007 |
| BN83250 | 320 | 63 | 1,000 | 28 | 0.28 | 100 | 0.09 | 100 | 0.0009 | <0.00009 |
| BN83470 | 270 | 56 | >1,00 0 | <10 | 0.22 | 100 | 0.08 | 100 | 0.0008 | <0.00008 |
| Valerolactam | 80 | 61 | >1,00 0 | <10 | 0.07 | 100 | 0.05 | 100 | 0.0009 | <0.00005 |

Although no information has previously been disclosed which would have led one skilled in the art to suppose that these functionally distinct classes of sstr2 agonist might exist, there are a few other examples of this phenomenon, where different agonists at the same receptor can cause different cellular responses, which is known as "functional selectivity" (see Kenakin (2007) Mol. Pharmacol. 72:1393-401 for an excellent recent review of this phenomenon at other receptors). A number of molecular mechanisms may underlie this phenomenon (such as active state-based selectivity, biased agonism, stimulus trafficking and so forth), but the nature of the underlying molecular mechanism is not germane to the ability to unambiguously distinguish the members of the groups by simple functional assays such as those illustrated here.

The groups of compounds used to illustrate here the two classes of sstr2 agonists are very distinct and readily distinguished (that is, all the members of each class are much more similar to each other than they are to any member of the other class, in terms of the functional responses they generate in several different assays). It is likely, however, that across all compound space there are agents that could be found which show "hybrid behaviour" - that is, they inhibit both GH/insulin secretion and chemokine-induced leukocyte migration with approximately equal potency and power. Such agents would not be readily assignable to either class on the basis of the methods provided here. As a result, we have provided a definition that unambiguously defines the members of each class, and excludes any such "hybrid behaviour" compounds from either category. Molecules with at least 2-fold higher potency (that is, the concentration required for half-maximal efficacy) in the GH or insulin secretion assay than in a chemokine-induced leukocyte migration assay (i.e. a ratio greater than 2 in the right hand columns of Table 3) are defined as "classical" sstr2 agonists. Conversely, molecules with at least 2-fold higher potency or power in the chemokine-induced leukocyte migration assay than in the GH or insulin secretion assay (i.e. a ratio less than 0.5 in the right hand columns of Table 3) are defined as "BSCI" sstr2 agonists (somatotaxins) according to the method of the present invention.

Note that it is preferable to compare assays using cells from the same species in performing the method of the invention. In the present example, where rat pituicytes are used to determine the effect of agents on classical sstr2 targets, the result is compared with the effect of the same agents on chemokine-induced migration of rat peripheral blood leukocytes. Similarly, where human Panc-2H4 cells are used to determine the effect of agents on classical sstr2 targets, the result is compared with the effect of the same agents on chemokine-induced migration of human THP-1 cells. Applying the method of the present invention across species risks mis-classifying compounds (in both directions) purely as a result of differing affinity for sstr2 from the two species. For example, a compound which had ten-fold lower affinity at rat sstr2 than human sstr2 would be misclassified as a "BSCI" sstr2 agonist by the method of the invention if the agent was three-fold lower potency in the rat pituicyte GH suppression assay than in the human THP-1 chemokine-induced leukocyte migration assay (because, when corrected for the 10-fold difference in binding affinity at the receptor, the "classical" activity was actually higher than the "BSCI" activity). For this reason, the ratio of "classical" to "BSCI" activity in Table 3 is calculated separately for rat and human.

### Example 8 : Identifying candidate BSCIs according to the method of the invention (1)

To illustrate further the method of the invention, it was applied to screen a series of beta-methyltryptophan containing sstr2 agonists related to L-779,976 for BSCI candidates. L-779,976 is one of the earliest non-peptide sstr2-selective agonists to be described (Rohrer et al. (1998) Science 282:737-40), and it was discovered through conventional screening, molecular design and medicinal chemistry approaches. It is loosely based around the key QFWK tetrapeptide (SEQ ID NO: 5) in the natural somatostatin ligand, and as a result exhibits classical sstr2 agonist properties (see Example 7).

Interestingly, the QxWK (SEQ ID NO: 6) was also identified as a key motif in the earliest peptide BSCIs (Fox et al. (2002) J. Med. Chem. 45:360-70), although the non-peptide BSCI sstr2 agonists described to date model the QxW end (see Fig. 1), whereas the classical sstr2 agonists model the WK end. This leads to the proposition that variants of L-779,976 fused with glutamine mimetic groupings may be a new class of BSCIs.

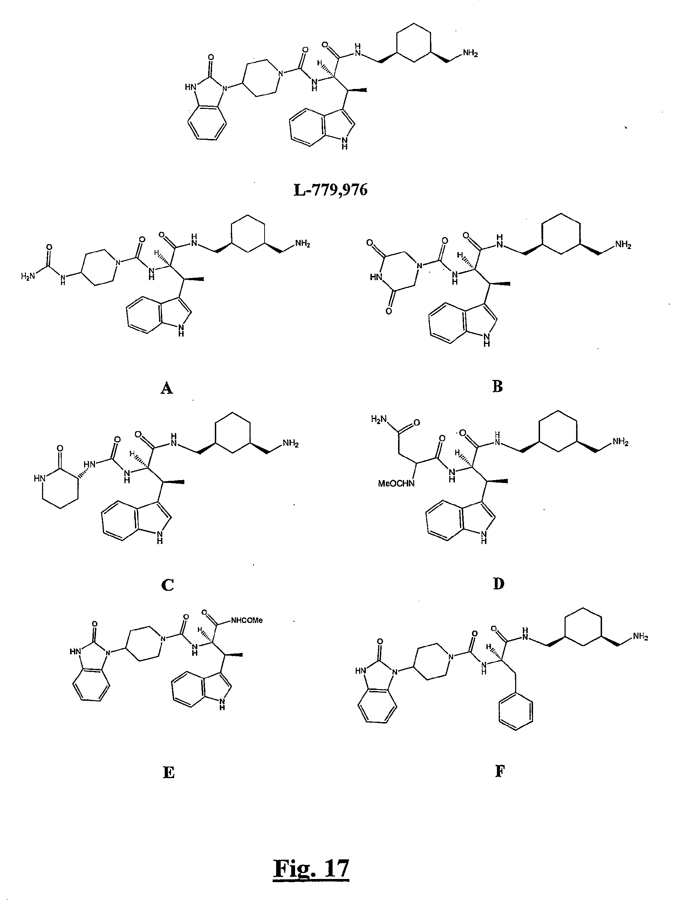

Here, we test half a dozen simple, novel L-779,976 derivatives according to the method of the invention, and demonstrate that one of them (designated "Compound C") is the first member of a novel structural class of BSCI compounds.
The six compounds, illustrated in Fig. 17, were subjected to the Panc-2H4 insulin suppression assay (exactly as described in Example 7) and the chemokine-induced THP-1 migration suppression assay (exactly as described in Example 1) at various concentrations from 0.01nM to 1,000nM in DMSO vehicle (1% final concentration). The results are shown in Table 4.

**Table 4. Classical and BSCI sstr2 agonist activity of six analogues of L-779,976. Suppression of glucose-dependent insulin secretion in Panc-2H4 cells was determined exactly as described in Example 7. Suppression of chemokine-induced THP-1 cell migration was determined exactly as described in Example 1. n.c. = ratio not calculated, for compounds showing insufficient activity in either assay.**

| **Compound** | **Panc-2H4 insulin suppression assay ("classical" target)** | | **Chemokine-induced THP-1 cell migration assay ("BSCI" target)** | | **RATIO** |
|---|---|---|---|---|---|
| | **ED50 (nM)** | **Max Effect (%)** | **ED50 (nM)** | **Max Effect (%)** | |
| L-779,976 | 17 | 40 | 450 | 65 | 26.5 |
| A | 109 | 38 | >1,000 | <10 | >9.1 |
| B | 167 | 52 | 220 | 100 | 1.3 |
| C | 82 | 56 | 2 | 100 | 0.02 |
| D | 650 | 37 | >1,000 | <10 | n.c. |
| E | >1,000 | <10 | >1,000 | <10 | n.c. |
| F | >1,000 | <10 | >1,000 | <10 | n.c. |

The parental L-779,976 and Compound A are classical sstr2 agonists. Compounds D, E and F have insufficient activity in either assay to be assessed according to the method of the present invention in this experiment. Compound B has both classical and BSCI sstr2 activity. However, the method of the invention identifies Compound C as a novel, structurally distinct BSCI compound (a somatotaxin), since the potency of suppression of chemokine-induced leukocyte migration is more than 2-fold higher than the potency of suppression of insulin secretion.

Here, we have illustrated how the method of the present invention has been applied to a series of analogues of a known sstr2 agonist compound to unambiguously identify the compound with BSCI activity, suitable for further development as an anti-inflammatory medicament. It is clear that this represents an application of the method of the invention, even though no experimental screening for binding to sstr2 was carried out because the knowledge of sstr2 binding was used in the first step to select the small number of molecules to be screened using the functional assays.

### Example 9 : Identifying candidate BSCIs according to the method of the invention (2)

In the previous example, knowledge of the molecular target of BSCIs was utilised to design candidate BSCI compounds for functional screening based on modification of a known sstr2 receptor agonist. However, the method of the invention can also be applied for random screening of very large libraries. In this application, a conventional molecular screen using sstr2 as the target is first applied to identify hits from the random library, and then in a second stage the hits (or analogues thereof) are screened using a pair of functional assays for "classical" sstr2 agonist activity and "BSCI" sstr2 agonist (somatotaxin) activity.

*Method:* A molecular screen for compounds interacting with sstr2 was established using membranes prepared from Jurkat T cells stably transfected to express sstr2 (Jurkat D3B; see Example 5). Briefly, Jurkat D3B cells (5 x10⁷ cells per preparation) were washed by centrifugation in Dulbecco's PBS and then resuspended in 1ml of ice-cold Dulbecco's PBS containing protease inhibitors (1mM PMSF, 100µM Leupeptin). The cell suspension was then lysed using manual Dounce homogenizer, and the resulting homogenate was spun at 700 x g for 1 minute at 4°C to pellet any unbroken cells and large cellular fragments. The supernatant was removed, and transferred to an ultracentrifuge tube and spun at 60,000 x g for 2 hours at 4°C to yield a pellet of membrane fragments. The pellet was gently washed with ice cold Dulbecco's PBS containing protease inhibitors, and then resuspended in 100µl of Dulbecco's PBS containing protease inhibitors. The protein concentration was then determined using a Coomassie Plus kit (Pierce) in accordance with the manufacturer's instructions and the final concentration was adjusted to 2mg/ml by addition of further Dulbecco's PBS containing protease inhibitors. An equal volume of glycerol was then added (yielding a final stock preparation of membranes at 1mg/ml total protein concentration in 50% glycerol) as a cryoprotectant and the stock was stored at -80°C for up to one month prior to use.

Screening was performed in 384-well microtitre plates. In each well a single reaction was set up consisting of 1µg Jurkat D3B membrane fraction protein in binding buffer (Gey's Balanced Salt Solution), 1nM [¹²⁵I]-labelled somatostatin-14 (400mCi/mmol) and the test compound from the library at 1mg/ml final concentration from 100mg/ml stock in DMSO, in a final reaction volume of 25µl. Binding was allowed to occur for 2 hours at 37°C (conditions previously established to result in equilibrium binding), and the reaction was then filtered through a GFC filtration sheet in a 384-well manifold, and was washed with 10ml) of binding buffer additionally containing 0.5% Triton X-100 and 300mM NaCl by continuous flow. Bound counts were assessed by quantitative scintigraphy. On each plate, BIM 23627 (10µM) was used as a positive control for binding and 100nM unlabelled somatostatin was used to determine the non-specific background. Binding was expressed as the percentage suppression of the specific counts in the presence of a given compound. Suppression of specific counts by 38% was the level four standard deviations below the negative (vehicle only) control level, assessed by running an entire 384-well plate through the assay without addition of any test compounds. As a result, test agents resulting in 76% or greater suppression of specific counts were designated as positive hits.

Hits from this assay were confirmed using conventional Scatchard analysis, exactly as described in Example 4 above. Compounds were considered for further evaluation if the apparent Ka for binding to sstr2 was 1µM or better.

Compounds with confirmed interaction at sstr2 were then evaluated in a pair of functional assays: the panc-2H4 insulin suppression assay (performed exactly as described in Example 7 as an estimate of "classical" sstr2 agonist activity) and the chemokine-induced THP-1 cell migration suppression assay (performed exactly as described in Example 1 as an estimate of "BSCI" sstr2 agonist activity). According to the method of the invention, agents with at least two-fold higher potency in the chemokine-induced THP-1 cell migration suppression assay are selected as candidate BSCIs suitable for further investigation and development as anti-inflammatory agents.

*Results:* Twenty-six assay plates were run (a total of 9,880 compounds screened), yielding 28 hits (0.28%) that suppressed the binding of labelled somatostatin-14 to sstr2 by more than 76%.

Of these primary hits, 11 (39% of the primary hits; 0.11% of the library) were successfully replicated in Scatchard analysis and 9 (81% of the replicated hits; 0.09% of the library) had an apparent binding affinity for sstr2 in excess of 1 µM.

The replicated hits (designated ASX.A through ASX.I in rank order of their apparent binding affinity for sstr2, with ASX.A having the highest apparent affinity) were then evaluated using the pair of functional assays, exactly as described in Example 7, and the results are shown in Table 5.

**Table 5. Classical and BSCI sstr2 agonist activity of the replicated moderate affinity (1µM or better) hits from the screening of 9,880 compounds. Suppression of glucose-dependent insulin secretion in Panc-2H4 cells was determined exactly as described in Example 7. Suppression of chemokine-induced THP-1 cell migration was determined exactly as described in Example 1. n.c. = ratio not calculated, for compounds showing insufficient activity in either assay. Data for L-779,976 and BN83250 are shown as examples of a classical-type and BSCI-type (somatotaixn) sstr2 agonist for comparison.**

| **Compound** | **Panc-2H4 insulin suppression assay ("classical" target)** | | **Chemokine-induced THP-1 cell migration assay ("BSCI" target)** | | **RATIO** |
|---|---|---|---|---|---|
| | **ED50 (nM)** | **Max Effect (%)** | **ED50 (nM)** | **Max Effect (%)** | |
| L-779,976 | 17 | 40 | 450 | 65 | 26.5 |
| BN83250 | 1,000 | 28 | 0.09 | 100 | <0.00009 |
| ASX.A | 180 | 52 | >1,000 | <10 | >5.6 |
| ASX.B | 680 | >50 | >1,000 | <1.0 | n.c. |
| ASX.C | 120 | 65 | 1,000 | 50 | 8.3 |
| ASX.D | 40 | 43 | 90 | 100 | 2.3 |
| ASX.E | >1,000 | <10 | >1,000 | <10 | n.c. |
| ASX.F | >1,000 | <10 | 720 | 30 | n.c. |
| ASX.G | >1,000 | <10 | >1,000 | <10 | n.c. |
| ASX.H | 1,000 | >20 | 45 | 100 | 0.045 |
| ASX.I | 550 | 48 | >1,000 | <10 | n.c. |

Surprisingly, 9 out of 11 compounds have detectable agonist activity in one or other of the functional assays. For most GPCRs, among hits identified by high throughput screening less than 10% (1 out of 10) would be expected to have agonist activity. This unusual finding may reflect the relative complexity of the agonist:receptor interaction at sstr2, or alternatively may be a consequence of the particular screen we chose to establish (competing for the binding of the natural ligand). In addition, the library screened was not random, but had been previously enriched with templates bearing structural similarity to known GPCR agonists.

Of these 9, three have significant agonist activity which can be clearly identified as "classical"-type sstr2 agonist activity (ASX.A, ASX.C and ASX.D) according to the method of the present invention, with at least 2-fold higher potency in the insulin suppression assay than in the cell migration suppression assay. By contrast, just one (ASX.H), which was among the lowest apparent binding affinities of the replicated hits, is unambiguously identified as a "BSCI"-type sstr2 agonist (a somatotaxin), with at least 2-fold higher potency in the cell migration suppression assay than in the insulin suppression assay. These findings illustrate that even the functional assay for suppression of cell migration cannot be used alone to unambiguously identify a "BSCI"-type sstr2 agonist (a somatotaxin), since one compound (ASX.D) had a comparable potency in this cell migration suppression assay to ASX.H, but had higher potency still in the insulin suppression assay for "classical" sstr2 agonist activity. The method of the present invention is both necessary and sufficient to identify entirely novel "BSCI"-type sstr2 agonists (somatotaxins).

Using the method of the present invention we screened 9,880 compounds to locate one which had adequate properties as a BSCI (although, of course, with an ED50 of 45nM it is some 1,000-fold less potent in vitro than the optimised lead candidates we have described previously; see, for example, WO2009/016390) and which could serve as a template for a conventional medicinal chemistry lead optimisation programme. Directly screening all 9,880 compounds through the functional assays would have taken many months (approximately 100 compounds can be screened in a normal working week). Application of the method of the present invention, exploiting the knowledge of the molecular target for BSCIs, allowed this entire model screening campaign to be completed in just one week - a reduction in the resources expended of some 99%. Typically, high throughput screens performed by the pharmaceutical industry might screen 10- to 100-fold more compounds than were used in this illustration. While such a screen would be straightforward using the method of the present invention, it would be wholly unachievable using the functional screening assays, which we estimate would have required almost a decade to achieve the same screening volume.

### Example 10 : In vivo validation of candidate BSCI identified according to the method of the invention

Compound P was identified according to the method of the invention, in order to indentify a pharmaceutically useful agent with anti-inflammatory properties but not the endocrine modulatory properties of classical somatostatin receptor agonists.

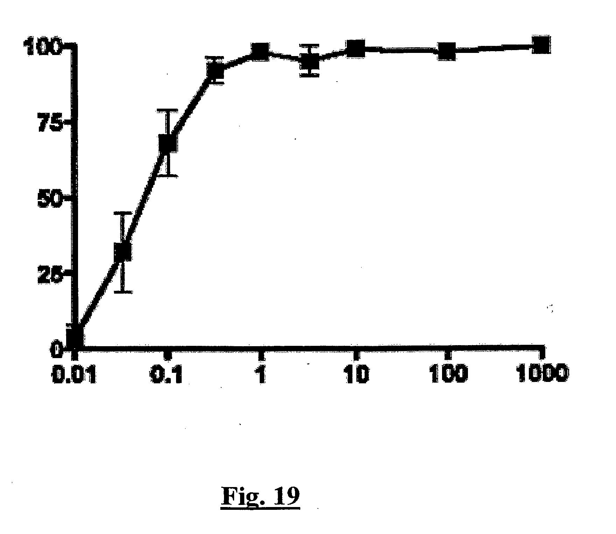

Compound P binds to sstr2, where it acts as an agonist. As shown in Fig. 19, Compound P inhibits the migration of THP-1 cells in response to the chemokine MCP-1, in an assay in accordance with the method of the invention, with an ED50 of approximately 0.05nM.

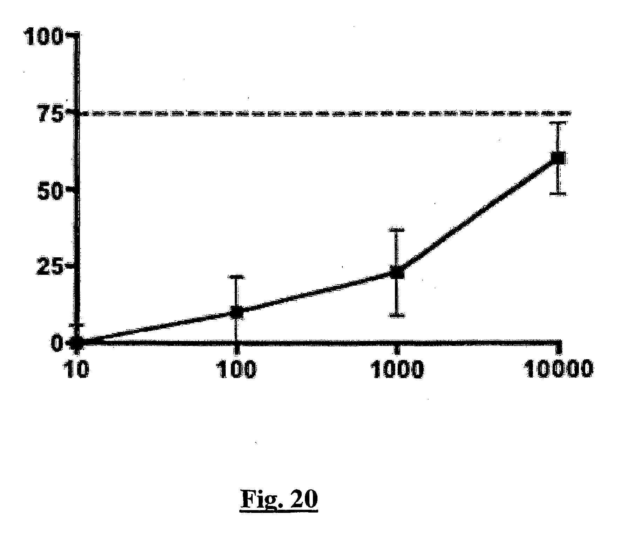

However, as shown in Fig. 20, Compound P only inhibited the release of Growth Hormone (GH) from cultured pituitary cells, in an assay performed in accordance with the method of the invention, with an ED50 of approximately 2000nM. In Fig. 20, the dotted line represents the maximal effect achievable in this assay, approximately 75% reduction in growth hormone secretion, with, for example, 10nM somatostatin-14, a classical sstr2 agonist. As a result, the ED50 dose is the dose of Compound P which reduced GH secretion by approximately 37%.

The ratio of classical-type activity to BSCI-type activity for Compound P is thus calculated to be approximately 0.000025.

In order to demonstrate that the assays performed in vitro were predictive of the activity of the compound in vivo, 36 normal, healthy human subjects of either gender were entered into a clinical study in three groups of 12 subjects. In each group of 12 subjects, 9 received Compound P via the oral route, as a liquid dose in 60mL of water, and the remaining 3 received only 60mL water as a placebo. The first group received 150mg of Compound P daily for 7 days, the second group received 500mg of Compound P daily for 7 days, and the third group received 1500mg of Compound P daily for 10 days. Blood samples were taken at various times before, during and after dosing with Compound P.

Compound P was found to have approximately 100% oral bioavailability in humans, with a half life of approximately 25 hours. The expected exposure of the subjects to Compound P was confirmed using a validated LC-MS assay for Compound P in the serum of the subjects.

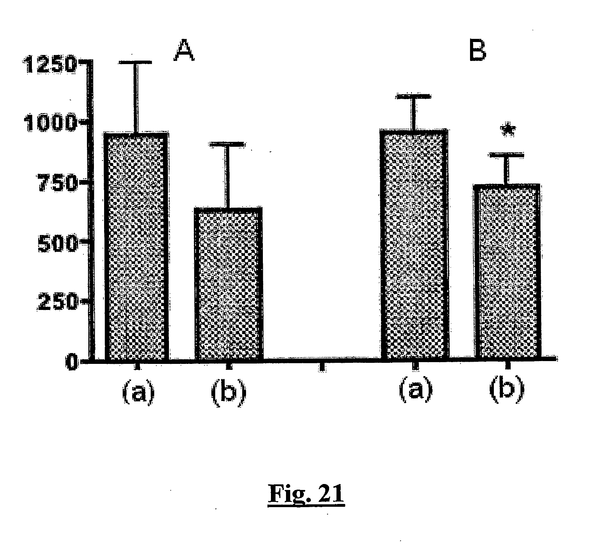

As expected, Compound P was an anti-inflammatory agent in man. C-reactive Protein (CRP) is a well accepted marker of the level of inflammation in man, and is elevated during acute and chronic inflammatory reactions (see Coventry et al. (2009) J. Translational Med. 7: 102). As a result, serum CRP levels are widely accepted as a surrogate marker for inflammation. CRP levels were measured using a commercially available ELISA assay in serum samples prior to dosing (pre-dose) and on the last day of dosing (post-dose). Treatment with Compound P at all three doses reduced the level of CRP in the treated subjects, but not in those who received the placebo. The effect of Compound P treatment (irrespective of dose) is shown in Fig. 21.

In Fig. 21, the effect of the well established, clinically anti-inflammatory therapeutic Medrol^{RTM} (methylprednisolone, a corticosteroid) and Compound P were compared. Both compounds lowered CRP to a similar extent, although the effect was only statistically significant for Compound P (p=0.02).

The results in Fig. 21 confirm that Compound P is at least as effective as an anti-inflammatory agent as the synthetic corticosteroid Medrol^{RTM}.

However, as also predicted according to the method of the invention, we have also found that Compound P had no effect on a panel of markers of the hypothalamic pituitary adrenal axis (HPA axis), despite binding to the type 2 somatostatin receptor, and therefore completely lack classical-type activity in man.

Compound P was found to have no effect on GH levels in humans treated for 7 days, even at very high doses. Furthermore, Compound P has no effect on ACTH levels in humans treated for 7 days, even at very high doses. ACTH is well established as the most sensitive marker of HPA axis disturbance in man (see Denef (2008) J. Neuroendocrinol. 20: 1-70), and taken together these investigations confirm that Compound P has clinically useful anti-inflammatory effects in man, while at the same doses having no classical-type somatostatin-mimetic effects.

This example confirms a compound identified according to the method of the present invention, which binds to the type 2 somatostatin receptor, has clinically useful anti-inflammatory activity in man in the complete absence of undesirable (in this context) endocrine-modulatory (or "classical-type") effects.

### Summary of the Examples

Taking the examples together, we have shown that BSCIs bind to the somatostatin receptor sstr2 and that this interaction is necessary and sufficient to confer sensitivity to BSCIs on leukocytes (Examples 1 through 5). However, not all sstr2 agonist are able to act as BSCIs (Example 6). As a result, we conclude that BSCIs represent a special, operationally defined sub-class of the larger general class of sstr2 receptor agonists (a class which we have termed "somatotaxins" since they are inhibitors of chemotaxis operating through a somatostatin receptor; Fig. 18). This sub-classification is useful (since it allows the identification of new BSCIs irrespective of any structural relationship to the known classes), novel (since it has never been described previously), well-defined (since a method is set out which unambiguously distinguishes members of the sub-class, illustrated in Examples 8 and 9) and surprising (since nothing in the existing scientific or patent literature suggests that functionally distinct sub-classes of sstr2 agonists might exist).

In both Example 8 and Example 9 prior knowledge of the molecular target for BSCIs, the subject of the present invention, was utilised in order to identify novel, structurally distinct BSCI compounds. In Example 8, the knowledge of the target was used to select L-779,976 as a suitable scaffold for analogue generation, and these analogues were subsequently screened using a pair of assays (one for "classical" sstr2 agonist activity and one for "BSCI" sstr2 agonist activity) according to the method of the invention in order to identify the most promising candidate for further development as an anti-inflammatory medicament. In Example 9, the knowledge of the target was used to design and deploy a straightforward molecular screen for sstr2 interacting structures, which were subsequently further refined through application of a pair of assays (one for "classical" sstr2 agonist activity and one for "BSCI" sstr2 agonist activity) according to the method of the invention. This resulted in the identification of the most promising library element for further development as an anti-inflammatory medicament.

Finally, in Example 10, the methods of the present invention were validated by demonstration that a compound identifiable by the invention, which binds to the type 2 somatostatin receptor, has clinically useful anti-inflammatory activity in man in the absence of undesirable endocrine-modulatory (or "classical-type") effects.

The surprising finding that BSCIs inhibit chemokine-induced cell migration by acting as agonists at the somatostatin type 2 receptor (sstr2), and furthermore that agents with BSCI activity are a special sub-class of sstr2 agonist, provides a unique and useful new way to identify agents with BSCI activity which have promise as novel anti-inflammatory agents for the treatment of a very wide range of diseases. Until now, the only way to identify novel, structurally distinct BSCI compounds was to modify the structure of existing BSCI compounds further (effectively expanding the number of known BSCI compounds within the existing structural classes) or to screen unselected compound libraries in the functional assays for BSCI activity (such as the Transwell^{RTM} cell migration assay used extensively in the examples for the present invention). Even the best technology available to day can only screen hundreds of new compounds through a functional assay, compared to the tens of thousands of compounds which can be passed through a conventional molecular screen in the same time period. This two to three orders of magnitude of difference in the processing rate through molecular and functional screens is key to the utility of the present invention: the resource implication of locating new structural classes of BSCI through random functional screening is impractically large. Using the method of the present invention, however, reduces the resource implication considerably (and renders the search practically achievable with current technology) by first performing a conventional molecular screen using the sstr2 receptor, and then in a second step further screening the hits from the molecular screen using functional assays to identify the functional signature of BSCIs (that is, agents with greater potency for the suppression of chemokine-induced leukocyte migration that for the suppression of GH or insulin release). Since the molecular screen in the first step reduces the number of compounds in a random library by two to three orders of magnitude (since reasonable potency hits typically represent only 0.1-1% of a random library), the resource requirements for the subsequent functional screens become practicable despite the higher resource utilisation per compound screened.

The sequences set out in the Sequence Listing below form part of the present description.

### Sequence Listing Free Text

Synthetic oligonucleotide (SEQ ID NOs 3-4);
Key BSCI motif of natural somatostatin ligand (SEQ ID NO: 5);
Key motif in early peptide BSCIs (SEQ ID NO: 6);
Xaa can be any naturally occurring amino acid (SEQ ID NO: 6).

## Claims

1. A method for the identification of a compound or agent with broad-spectrum chemokine inhibitor (BSCI) activity comprising the following steps:
(a) firstly one or more candidate compounds or agents other than somatostatin are screened for binding to a somatostatin receptor, for example the type 2 somatostatin receptor (sstr2) such as sstr2A or sstr2B;
then (b) compounds or agents which show binding to the somatostatin receptor are tested for BSCI activity in a functional assay,
wherein the method excludes treatment of a human or animal body by surgery or therapy.

2. The method of claim 1, comprising the following further step:
(c) compounds or agents which show binding to the somatostatin receptor are tested for classical sstr2 agonist activity in a functional assay,
wherein step (a) is completed first, but steps (b) and (c) are performed in any order, or simultaneously.

3. The method of claim 1 or claim 2 wherein the functional assay for BSCI activity is an assay for the suppression of chemokine-induced cell migration in vitro or in vivo.

4. The method of any preceding claim wherein the functional assay for BSCI activity is an assay for the cytokine secretion of T helper cells.

5. The method of any preceding claim wherein the candidate compounds or agents to be screened are selected from, or together compose, a library, and/or are produced by combinatorial chemistry.

6. The method of any preceding claim wherein the candidate agents to be tested are mixtures of compounds.

7. The method of any preceding claim wherein molecular interaction with the somatostatin receptor is performed using whole cells, or membranes from cells, expressing the somatostatin receptor.

8. The method of claim 7 wherein the cells have been engineered to over-express the somatostatin receptor, and/or wherein the cells express, or have been engineered to express, a functional fragment thereof that retains the ability to bind to the natural ligand or another ligand of the somatostatin receptor.

9. The method of any of claims 1-6 wherein molecular interaction with the somatostatin receptor is performed using the isolated or purified somatostatin receptor or any functional fragment thereof that retains the ability to bind to the natural ligand or another ligand of the somatostatin receptor.

10. The method of any preceding claim wherein interaction between the candidate compounds or agents is determined by competition with another labelled ligand capable of binding to the somatostatin receptor.

11. The method of any of claims 1-9 wherein interaction between the candidate compounds or agents is determined directly.

12. The method of any preceding claim wherein interaction of the candidate compounds or agents with the somatostatin receptor is determined or inferred by interrogation of the scientific literature or pre-existing databases of any kind.

13. The method of any of claim 2 or claims 3-12 when dependent on claim 2 wherein the functional assay for classical sstr2 agonist activity is suppression of pituitary hormone (such as growth hormone or thyroid stimulating hormone) release.

14. The method of any of claim 2 or claims 3-12 when dependent on claim 2 wherein the functional assay for classical sstr2 agonist activity is modulation of glucose handling, for example detected by measuring insulin secretion or by measuring glucagon secretion.

15. The method of any of claim 2 or claims 3-14 when dependent on claim 2 wherein the assay is performed in vitro or in vivo.

16. The method of claim 15 wherein the assay is performed in vivo and a surrogate biomarker is used to detect the consequences of classical sstr2 agonist activity.

17. The method of any preceding claim with an additional step to define a novel structural class of somatotaxins based on one or more structural motifs present within the compounds selected through application of the prior steps.

## Patentansprüche

1. Verfahren zum Identifizieren einer Verbindung oder eines Agens mit Breitspektrum-Chemokin-Inhibitor-(BSCI)-Aktivität, das die folgenden Schritte beinhaltet:
(a) zunächst Screenen von einer oder mehreren Kandidatverbindungen oder -agenzien außer Somatostatin im Hinblick auf eine Bindung an einen Somatostatinrezeptor, z.B. den Typ-2-Somatostatin-Rezeptor (sstr2) wie sstr2A oder sstr2B;
(b) dann Testen von Verbindungen oder Agenzien, die eine Bindung an den Somatostatin-Rezeptor aufweisen, im Hinblick auf BSCI-Aktivität in einem Funktionsassay,
wobei das Verfahren die Behandlung eines menschlichen oder tierischen Körpers durch Chirurgie oder Therapie ausschließt.

2. Verfahren nach Anspruch 1, das den folgenden weiteren Schritt beinhaltet:
(c) Testen von Verbindungen oder Agenzien, die eine Bindung an den Somatostatin-Rezeptor aufweisen, im Hinblick auf eine klassische sstr2-Agonistenaktivität in einem Funktionsassay,
wobei Schritt (a) zuerst abgeschlossen wird, die Schritte (b) und (c) jedoch in einer beliebigen Reihenfolge oder gleichzeitig durchgeführt werden.

3. Verfahren nach Anspruch 1 oder Anspruch 2, wobei der Funktionsassay im Hinblick auf BSCI-Aktivität ein Assay für die Unterdrückung der Chemokin-induzierten Zellmigration in vitro oder in vivo ist.

4. Verfahren nach einem vorherigen Anspruch, wobei der Funktionsassay im Hinblick auf BSCI-Aktivität ein Assay für die Zytokinsekretion von T-Helferzellen ist.

5. Verfahren nach einem vorherigen Anspruch, wobei die zu screenenden Kandidatverbindungen oder -agenzien aus einer Bibliothek ausgewählt werden oder zusammen eine solche bilden und/oder durch kombinatorische Chemie produziert werden.

6. Verfahren nach einem vorherigen Anspruch, wobei die zu testenden Kandidatagenzien Gemische aus Verbindungen sind.

7. Verfahren nach einem vorherigen Anspruch, wobei die molekulare Interaktion mit dem Somatostatin-Rezeptor unter Verwendung von ganzen Zellen oder Membranen von Zellen erfolgt, die den Somatostatin-Rezeptor exprimieren.

8. Verfahren nach Anspruch 7, wobei die Zellen so konstruiert wurden, dass sie den Somatostatin-Rezeptor überexprimieren, und/oder wobei die Zellen ein funktionelles Fragment davon, das die Fähigkeit beibehält, sich an den natürlichen Liganden oder einen anderen Liganden des Somatostatin-Rezeptors zu binden, exprimieren oder zum Exprimieren eines solchen konstruiert wurden.

9. Verfahren nach einem der Ansprüche 1-6, wobei die molekulare Interaktion mit dem Somatostatin-Rezeptor unter Verwendung des isolierten oder gereinigten Somatostatin-Rezeptors oder eines beliebigen funktionellen Fragments davon erfolgt, das die Fähigkeit beibehält, sich an den natürlichen Liganden oder einen anderen Liganden des Somatostatin-Rezeptors zu binden.

10. Verfahren nach einem vorherigen Anspruch, wobei die Interaktion zwischen den Kandidatverbindungen oder-agenzien durch die Konkurrenz mit einem anderen markierten Liganden bestimmt wird, der sich an den Somatostatin-Rezeptor binden kann.

11. Verfahren nach einem der Ansprüche 1-9, wobei die Interaktion zwischen den Kandidatverbindungen oder-agenzien direkt bestimmt wird.

12. Verfahren nach einem vorherigen Anspruch, wobei die Interaktion der Kandidatverbindungen oder -agenzien mit dem Somatostatin-Rezeptor durch Hinzuziehen wissenschaftlicher Literatur oder vorher existierender Datenbanken jeglicher Art bestimmt oder hergeleitet wird.

13. Verfahren nach einem der Ansprüche 2 oder 3-12, in Abhängigkeit von Anspruch 2, wobei der Funktionsassay für klassische sstr2-Agonisten-Aktivität die Unterdrückung der Hypophysenhormon-(wie Wachstumshormon oder thyreoidstimulierendes Hormon)-Freisetzung ist.

14. Verfahren nach einem der Ansprüche 2 oder 3-12, in Abhängigkeit von Anspruch 2, wobei der Funktionsassay für klassische sstr2-Agonisten-Aktivität die Modulation der Glucosehandhabung ist, die beispielsweise durch Messen der Insulinsekretion oder durch Messen der Glucagonsekretion detektiert wird.

15. Verfahren nach einem der Ansprüche 2 oder 3-14, in Abhängigkeit von Anspruch 2, wobei der Assay in vitro oder in vivo durchgeführt wird.

16. Verfahren nach Anspruch 15, wobei der Assay in vivo durchgeführt wird und ein Surrogat-Biomarker zum Detektieren der Auswirkungen der klassischen sstr2-Agonisten-Aktivität verwendet wird.

17. Verfahren nach einem vorherigen Anspruch mit einem zusätzlichen Schritt des Definierens einer neuen Strukturklasse von Somatotaxinen auf der Basis von einem oder mehreren Strukturmotiven, die in den Verbindungen vorliegen, die durch Anwenden der vorherigen Schritte ausgewählt werden.

## Revendications

1. Méthode d'identification d'un composé ou agent présentant une activité inhibitrice des chimiokines à large spectre (BSCI), qui comprend les étapes suivantes :
(a) un ou plusieurs composés ou agents candidats autres que la somatostatine sont tout d'abord triés en fonction de leur capacité de liaison à un récepteur à la somatostatine, par exemple au récepteur à la somatostatine de type 2 (sstr2) comme sstr2A ou sstr2B ;
puis (b) l'activité BSCI des composés ou agents qui se lient au récepteur à la somatostatine est testée lors d'un essai fonctionnel,
ladite méthode excluant le traitement d'un corps humain ou animal par chirurgie ou thérapie.

2. Méthode de la revendication 1, qui comprend l'étape additionnelle suivante :
(c) l'activité agoniste du sstr2 classique des composés ou agents qui se lient au récepteur à la somatostatine est testée lors d'un essai fonctionnel,
dans laquelle l'étape (a) est effectuée en premier mais les étapes (b) et (c) sont réalisées dans tout ordre ou simultanément.

3. Méthode de la revendication 1 ou de la revendication 2, dans laquelle l'essai fonctionnel de l'activité BSCI est un essai de la suppression de la migration cellulaire induite par les chimiokines *in vitro* ou *in vivo.*

4. Méthode de l'une quelconque des revendications précédentes, dans laquelle l'essai fonctionnel de l'activité BSCI est un essai de la sécrétion de cytokines par les cellules T auxiliaires.

5. Méthode de l'une quelconque des revendications précédentes, dans laquelle les composés ou agents candidats à trier sont sélectionnés dans, ou forment ensemble, une librairie et/ou sont produits par chimie combinatoire.

6. Méthode de l'une quelconque des revendications précédentes, dans laquelle les agents candidats à tester sont des mélanges de composés.

7. Méthode de l'une quelconque des revendications précédentes, dans laquelle une interaction moléculaire avec le récepteur à la somatostatine est effectuée en utilisant des cellules entières ou des membranes de cellules exprimant le récepteur à la somatostatine.

8. Méthode de la revendication 7, dans laquelle les cellules ont été modifiées de manière à surexprimer le récepteur à la somatostatine et/ou dans laquelle les cellules expriment, ou ont été modifiées de manière à exprimer, un fragment fonctionnel de celui-ci qui conserve la capacité à se lier au ligand naturel ou à un autre ligand du récepteur à la somatostatine.

9. Méthode de l'une quelconque des revendications 1-6, dans laquelle l'interaction moléculaire avec le récepteur à la somatostatine est effectuée en utilisant un récepteur à la somatostatine isolé ou purifié ou tout fragment fonctionnel de celui-ci qui conserve la capacité à se lier au ligand naturel ou à un autre ligand du récepteur à la somatostatine.

10. Méthode de l'une quelconque des revendications précédentes, dans laquelle l'interaction entre les composés ou agents candidats est déterminée par compétition avec un autre ligand marqué capable de se lier au récepteur à la somatostatine.

11. Méthode de l'une quelconque des revendications 1-9, dans laquelle l'interaction entre les composés ou agents candidats est déterminée directement.

12. Méthode de l'une quelconque des revendications précédentes, dans laquelle l'interaction des composés ou agents candidats avec le récepteur à la somatostatine est déterminée ou déduite par une interrogation de la littérature scientifique ou de bases de données préexistantes de tout type.

13. Méthode de l'une quelconque de la revendication 2 ou des revendications 3-12 quand dépendantes de la revendication 2, dans laquelle l'essai fonctionnel de l'activité agoniste du sstr2 classique est la suppression de la libération d'une hormone hypophysaire (comme l'hormone de croissance ou la thyréostimuline).

14. Méthode de l'une quelconque de la revendication 2 ou des revendications 3-12 quand dépendantes de la revendication 2, dans laquelle l'essai fonctionnel de l'activité agoniste du sstr2 classique est la modulation de la gestion du glucose détectée par exemple par une mesure de la sécrétion d'insuline ou une mesure de la sécrétion de glucagon.

15. Méthode de l'une quelconque de la revendication 2 ou des revendications 3-14 quand dépendantes de la revendication 2, dans laquelle l'essai est effectué *in vitro* ou *in vivo.*

16. Méthode de la revendication 15, dans laquelle l'essai est effectué *in vivo* et un biomarqueur de substitution est utilisé pour détecter les conséquences de l'activité agoniste du sstr2 classique.

17. Méthode de l'une quelconque des revendications précédentes, qui comprend une étape additionnelle pour définir une nouvelle classe structurelle de « somatotaxines » sur la base d'un ou de plusieurs motifs structuraux présents dans les composés sélectionnés lors de la réalisation des étapes antérieures.


### Sequence Listing

<110> Cambridge Enterprise Limited
<120> Improved Methods for Identification
<130> P/63515.WO01
<150> GB 0903495.0
   <151> 2009-02-27
<150> US 12/394,897
   <151> 2009-02-27
<160> 6
<170> Patent In version 3.5
<210> 1
   <211> 2996
   <212> DNA
   <213> Homo sapiens
<220>
   <221> CDS
   <222> (361)..(1470)
<400> 1
   cgcagccacc catgcgcgcg cgctcgcaag accaccagcg cccagagccc cagtctgagg      60
   cttggcgccg ggggtctgcg ggcgagggga gctctctacg tgcgaggggc tagcgggagc      120
   cggcacaaga gggtcgagga gccaggaacc ccaaacgtcc ggcgccaggc gctagccaag      180
   ctgctgcgcg ccccggcgcc cagctggctc ggggacagcc gctgggtgtc ggagaccgga      240
   gctagcggat tgcagcggaa aagcaaagat gtcacactgg atccttggcc tccagggtcc      300
   attaaggtga gaataagatc tctgggctgg ctggaactag cctaagactg aaaagcagcc      360
   atg gac atg gcg gat gag cca ctc aat gga agc cac aca tgg cta tcc        408
   Met Asp Met Ala Asp Glu Pro Leu Asn Gly Ser His Thr Trp Leu Ser
   1               5                   10                  15
   att cca ttt gac ctc aat ggc tct gtg gtg tca acc aac acc tca aac        456
   Ile Pro Phe Asp Leu Asn Gly Ser Val Val Ser Thr Asn Thr Ser Asn
               20                  25                  30
   cag aca gag ccg tac tat gac ctg aca agc aat gca gtc ctc aca ttc        504
   Gln Thr Glu Pro Tyr Tyr Asp Leu Thr Ser Asn Ala Val Leu Thr Phe
           35                  40                  45
   atc tat ttt gtg gtc tgc atc att ggg ttg tgt ggc aac aca ctt gtc        552
   Ile Tyr Phe Val Val Cys Ile Ile Gly Leu Cys Gly Asn Thr Leu Val
       50                  55                  60
   att tat gtc atc ctc cgc tat gcc aag atg aag acc atc acc aac att      600
   Ile Tyr Val Ile Leu Arg Tyr Ala Lys Met Lys Thr Ile Thr Asn Ile
   65                  70                  75                  80
   tac atc ctc aac ctg gcc atc gca gat gag ctc ttc atg ctg ggt ctg      648
   Tyr Ile Leu Asn Leu Ala Ile Ala Asp Glu Leu Phe Met Leu Gly Leu
                   85                  90                  95
   cct ttc ttg gct atg cag gtg gct ctg gtc cac tgg ccc ttt ggc aag      696
   Pro Phe Leu Ala Met Gln Val Ala Leu Val His Trp Pro Phe Gly Lys
               100                 105                 110
   gcc att tgc cgg gtg gtc atg act gtg gat ggc atc aat cag ttc acc      744
   Ala Ile Cys Arg Val Val Met Thr Val Asp Gly Ile Asn Gln Phe Thr
           115                 120                 125
   agc atc ttc tgc ctg aca gtc atg agc atc gac cga tac ctg gct gtg      792
   Ser Ile Phe Cys Leu Thr Val Met Ser Ile Asp Arg Tyr Leu Ala Val
       130                 135                 140
   gtc cac ccc atc aag tcg gcc aag tgg agg aga ccc cgg acg gcc aag      840
   Val His Pro Ile Lys Ser Ala Lys Trp Arg Arg Pro Arg Thr Ala Lys
   145                 150                 155                 160
   atg atc acc atg gct gtg tgg gga gtc tct ctg ctg gtc atc ttg ccc      888
   Met Ile Thr Met Ala Val Trp Gly Val Ser Leu Leu Val Ile Leu Pro
                   165                 170                 175
   atc atg ata tat gct ggg ctc cgg agc aac cag tgg ggg aga agc agc      936
   Ile Met Ile Tyr Ala Gly Leu Arg Ser Asn Gln Trp Gly Arg Ser Ser
               180                 185                 190
   tgc acc atc aac tgg cca ggt gaa tct ggg gct tgg tac aca ggg ttc      984
   Cys Thr Ile Asn Trp Pro Gly Glu Ser Gly Ala Trp Tyr Thr Gly Phe
           195                 200                 205
   atc atc tac act ttc att ctg ggg ttc ctg gta ccc ctc acc atc atc     1032
   Ile Ile Tyr Thr Phe Ile Leu Gly Phe Leu Val Pro Leu Thr Ile Ile
       210                 215                 220
   tgt ctt tgc tac ctg ttc att atc atc aag gtg aag tcc tct gga atc     1080
   Cys Leu Cys Tyr Leu Phe Ile Ile Ile Lys Val Lys Ser Ser Gly Ile
   225                 230                 235                 240
   cga gtg ggc tcc tct aag agg aag aag tct gag aag aag gtc acc cga     1128
   Arg Val Gly Ser Ser Lys Arg Lys Lys Ser Glu Lys Lys Val Thr Arg
                   245                 250                 255
   atg gtg tcc atc gtg gtg gct gtc ttc atc ttc tgc tgg ctt ccc ttc     1176
   Met Val Ser Ile Val Val Ala Val Phe Ile Phe Cys Trp Leu Pro Phe
               260                 265                 270
   tac ata ttc aac gtt tct tcc gtc tcc atg gcc atc agc ccc acc cca     1224
   Tyr Ile Phe Asn Val Ser Ser Val Ser Met Ala Ile Ser Pro Thr Pro
           275                 280                 285
   gcc ctt aaa ggc atg ttt gac ttt gtg gtg gtc ctc acc tat gct aac     1272
   Ala Leu Lys Gly Met Phe Asp Phe Val Val Val Leu Thr Tyr Ala Asn
       290                 295                 300
   agc tgt gcc aac cct atc cta tat gcc ttc ttg tct gac aac ttc aag      1320
   Ser Cys Ala Asn Pro Ile Leu Tyr Ala Phe Leu Ser Asp Asn Phe Lys
   305                 310                 315                 320
   aag agc ttc cag aat gtc ctc tgc ttg gtc aag gtg agc ggc aca gat      1368
   Lys Ser Phe Gln Asn Val Leu Cys Leu Val Lys Val Ser Gly Thr Asp
               325                 330                 335
   gat ggg gag cgg agt gac agt aag cag gac aaa tcc cgg ctg aat gag      1416
   Asp Gly Glu Arg Ser Asp Ser Lys Gln Asp Lys Ser Arg Leu Asn Glu
           340                 345                 350
   acc acg gag acc cag agg acc ctc ctc aat gga gac ctc caa acc agt      1464
   Thr Thr Glu Thr Gln Arg Thr Leu Leu Asn Gly Asp Leu Gln Thr Ser
       355                 360                 365
   atc tga actgcttggg gggtgggaaa gaaccaagcc atgctctgtc tactggcaat       1520
   Ile
   gggctcccta cccacactgg cttcctgcct cccacccctc acacctggct tctagaatag    1580
   aggattgctc agcatgagtc caattcagag aacggtgttt gagtcagctt gtctgattga    1640
   atgataatgt gctaaattga ttacctcccc cttaaagcga acactgaaat gcaggtagac    1700
   aattcaaagt ctggagaaga gggatcatgc ctggatatga tctttagaaa caacaaaaat    1760
   agaaaaaaat aagtatctgt gtgtttgtgt attgaaaact caatatgtaa tcttgtgttt    1820
   ttatatgtat acttgtatat tcctatttat tctctgtata ggcattacct acgttcctgt    1880
   gtttacatac acaagtagca aattcgagta tgcatagtgt agatggacat ttgccacaac    1940
   acactgcccg cagaaatgga cttaccgtga agccaataaa gttcaagctt cagggatctc    2000
   tcttgcacgg gccttgccaa ggcccaggag ggacttgggc agtatgttca tgtggtcata    2060
   tgtttttgta aaaaattgtg aaagtaagat atgtttgtat tgtttttctt aaagaggaac    2120
   ctcgtataag cttcaagcct cacaaacctt ctagcctctg cccttgggga tttgcttcat    2180
   taatttcagg caagtgaggt caatgtaaga agggaaaggg agaagatatt tgaagaacca    2240
   gaatgtaaat tcatgtgttt ccacttctca gatatagtca gagaattatt catttgccca    2300
   aaaggactta agtggttgtg gtcatccatc attgtattta tcaagacaaa gccaactttg    2360
   ttataagatt gcattttttt cttttcaaat tgctttagtt tttcttaggg agctatgagg    2420
   gggaaaaatc actaacatga aaggcaaaaa atggactatg attcctgtgg ggaaacaatt    2480
   tcattctctc catcgtgaaa ataagtgaat aagagtgaag caaaattaca cctttatgag    2540
   aaaccataaa attgttttta tttttcaggc cagacatagc ttcctaatga aagaaaatgg    2600
   aaatgtaatt cgacgactcc tcaaagggga ctttagagga cttcatacaa agctgggcat      2660
   taagaaaacc acaatgcatg gccgggcgtg gtggcttaca cctgtaatcc cagcactttg      2720
   ggaggccgag gtgggtggat cacccgaggt caggagttcg agaccagcct ggccaacatg      2780
   gtgaaacccc atcactacta aaaatatgta aattagtcgg gcgtggtgtc acgtgcctgt      2840
   aatcctagct gctcgggagg ctgaggcagg agaatcactt gaacttggga ggtggaggtt      2900
   gcagtaagct gagattgtgc cactgcactc tagcctgagc aacaagagca aaactcagtc      2960
   tcaaaaaaaa aaaaaaaaaa aaaaaaaaaa aaaaaa                                2996
<210> 2
   <211> 369
   <212> PRT
   <213> Homo sapiens
<400> 2
   Met Asp Met Ala Asp Glu Pro Leu Asn Gly Ser His Thr Trp Leu Ser
   1               5                   10                  15
   Ile Pro Phe Asp Leu Asn Gly Ser Val Val Ser Thr Asn Thr Ser Asn
               20                  25                  30
   Gln Thr Glu Pro Tyr Tyr Asp Leu Thr Ser Asn Ala Val Leu Thr Phe
           35                  40                  45
   Ile Tyr Phe Val Val Cys Ile Ile Gly Leu Cys Gly Asn Thr Leu Val
       50                  55                  60
   Ile Tyr Val Ile Leu Arg Tyr Ala Lys Met Lys Thr Ile Thr Asn Ile
   65                  70                  75                  80
   Tyr Ile Leu Asn Leu Ala Ile Ala Asp Glu Leu Phe Met Leu Gly Leu
                   85                  90                  95
   Pro Phe Leu Ala Met Gln Val Ala Leu Val His Trp Pro Phe Gly Lys
               100                 105                 110
   Ala Ile Cys Arg Val Val Met Thr Val Asp Gly Ile Asn Gln Phe Thr
           115                 120                 125
   Ser Ile Phe Cys Leu Thr Val Met Ser Ile Asp Arg Tyr Leu Ala Val
       130                 135                 140
   Val His Pro Ile Lys Ser Ala Lys Trp Arg Arg Pro Arg Thr Ala Lys
   145 150 155 160
   Met Ile Thr Met Ala Val Trp Gly Val Ser Leu Leu Val Ile Leu Pro
   165 170 175
   Ile Met Ile Tyr Ala Gly Leu Arg Ser Asn Gln Trp Gly Arg Ser Ser
   180 185 190
   Cys Thr Ile Asn Trp Pro Gly Glu Ser Gly Ala Trp Tyr Thr Gly Phe
   195 200 205
   Ile Ile Tyr Thr Phe Ile Leu Gly Phe Leu Val Pro Leu Thr Ile Ile
   210 215 220
   Cys Leu Cys Tyr Leu Phe Ile Ile Ile Lys Val Lys Ser Ser Gly Ile
   225 230 235 240
   Arg Val Gly Ser Ser Lys Arg Lys Lys Ser Glu Lys Lys Val Thr Arg
   245 250 255
   Met Val Ser Ile Val Val Ala Val Phe Ile Phe Cys Trp Leu Pro Phe
   260 265 270
   Tyr Ile Phe Asn Val Ser Ser Val Ser Met Ala Ile Ser Pro Thr Pro
   275 280 285
   Ala Leu Lys Gly Met Phe Asp Phe Val Val Val Leu Thr Tyr Ala Asn
   290 295 300
   Ser Cys Ala Asn Pro Ile Leu Tyr Ala Phe Leu Ser Asp Asn Phe Lys
   305 310 315 320
   Lys Ser Phe Gln Asn Val Leu Cys Leu Val Lys Val Ser Gly Thr Asp
   325 330 335
   Asp Gly Glu Arg Ser Asp Ser Lys Gln Asp Lys Ser Arg Leu Asn Glu
   340 345 350
   Thr Thr Glu Thr Gln Arg Thr Leu Leu Asn Gly Asp Leu Gln Thr Ser
   355 360 365
   Ile
<210> 3
   <211> 20
   <212> DNA
   <213> Artificial Sequence
<220>
   <223> Synthetic oligonucleotide
<400> 3
   gccaagatga agaccatcac 20
<210> 4
   <211> 21
   <212> DNA
   <213> Artificial Sequence
<220>
   <223> Synthetic oligonucleotide
<400> 4
   gatgaaccct gtgtaccaag c 21
<210> 5
   <211> 4
   <212> PRT
   <213> Artificial Sequence
<220>
   <223> Key BSCI motif of natural somatostatin ligand
<400> 5
   Gln Phe Trp Lys
   1
<210> 6
   <211> 4
   <212> PRT
   <213> Artificial Sequence
<220>
   <223> Key motif in early peptide BSCIs
<220>
   <221> misc_feature
   <222> (2)..(2)
   <223> Xaa can be any naturally occurring amino acid
<400> 6
   Gln Xaa Trp Lys
   1